# EUROPÄISCHE PATENTSCHRIFT

(11) **EP 1 918 364 B1**
(45) Veröffentlichungstag und Bekanntmachung des Hinweises auf die Patenterteilung: **06.07.2011**
(21) Anmeldenummer: 08151018.2
(22) Anmeldetag: 17.08.2004
(51) Int. Cl.: C12N 5/09, C12N 5/071

(54) **Verfahren zur Reklonierung von Produktionszellen**
Method for recloning production cells
Procedé de reclonage de cellules de production

(30) Priorität: 19.08.2003 DE 10338531
(43) Veröffentlichungstag der Anmeldung: 07.05.2008
(62) Teilanmeldung aus: 04764194.9
(73) Patentinhaber: Boehringer Ingelheim Pharma GmbH & Co. KG, 55216 Ingelheim am Rhein (DE)
(72) Erfinder: Enenkel, Barbara, Dr., 88447 Warthausen (DE); Fieder, Jürgen, 89619 Unterstadion (DE); Otto, Ralf, Dr., 88422 Oggelshausen (DE); Krieg, Thomas, 88444 Ummendorf (DE)
(74) Vertreter: Hammann, Heinz

(56) Entgegenhaltungen:
- WO-A-02/29012
- US-A1- 2003 032 182
- US-A1- 2003 073 234
- HUGIN A W: "Cloning of cells with autologous feeder cells" JOURNAL OF IMMUNOLOGICAL METHODS, ELSEVIER SCIENCE PUBLISHERS B.V.,AMSTERDAM, NL, Bd. 205, Nr. 2, 14. Juli 1997 (1997-07-14), Seiten 211-212, XP004126294 ISSN: 0022-1759
- BREZINSKY S C G ET AL: "A simple method for enriching populations of transfected CHO cells for cells of higher specific productivity" JOURNAL OF IMMUNOLOGICAL METHODS, ELSEVIER SCIENCE PUBLISHERS B.V.,AMSTERDAM, NL, Bd. 277, Nr. 1-2, 1. Juni 2003 (2003-06-01), Seiten 141-155, XP004430554 ISSN: 0022-1759
- BATTYE F L ET AL: "Single cell sorting and cloning" JOURNAL OF IMMUNOLOGICAL METHODS, ELSEVIER SCIENCE PUBLISHERS B.V.,AMSTERDAM, NL, Bd. 243, Nr. 1-2, 21. September 2000 (2000-09-21), Seiten 25-32, XP004210690 ISSN: 0022-1759
- BORTH N ET AL: "Efficient selection of high-producing subclones during gene amplification of recombinant Chinese hamster ovary cells by flow cytometry and cell sorting." BIOTECHNOLOGY AND BIOENGINEERING. 2000-2001, Bd. 71, Nr. 4, 2000, Seiten 266-273, XP002317361 ISSN: 0006-3592
- ECHCHAKIR H ET AL: "Evidence for in situ expansion of diverse antitumor-specific cytotoxic T lymphocyte clones in a human large cell carcinoma of the lung." INTERNATIONAL IMMUNOLOGY. APR 2000, Bd. 12, Nr. 4, April 2000 (2000-04), Seiten 537-546, XP002317362 ISSN: 0953-8178
- HLINAK A ET AL: "Feeder cells from different sources and conditioned media for recloning of human--mouse and mouse--mouse hybridomas." FOLIA BIOLOGICA. 1988, Bd. 34, Nr. 2, 1988, Seiten 105-117, XP008042737 ISSN: 0015-5500
- BORRELLI M J ET AL: "EVIDENCE THAT THE FEEDER EFFECT IN MAMMALIAN CELLS IS MEDIATED BY A DIFFUSIBLE SUBSTANCE" INTERNATIONAL JOURNAL OF HYPERTHERMIA, Bd. 5, Nr. 1, 1989, Seiten 99-104, XP008042840 ISSN: 0265-6736
- GREEN BRONWYN J ET AL: "Rapid screening for high-titer retroviral packaging cell lines using an in situ fluorescence assay." HUMAN GENE THERAPY. 10 JUN 2002, Bd. 13, Nr. 9, 10. Juni 2002 (2002-06-10), Seiten 1005-1013, XP002333721 ISSN: 1043-0342

## Beschreibung

### Gebiet der Erfindung

Die vorliegende Erfindung bezieht sich auf das Gebiet der Zellkulturtechnologie und betrifft die Verwendung einer Hamsterzelle als feeder-Zelle zur Vermehrung/Klonierung von in Suspension wachsenden autologen Hamster Zellen unter serumfreien Bedingungen.

### Hintergrund der Erfindung

Zur biotechnologischen Herstellung von biologisch aktiven bzw. therapeutischen Proteinen in Säugerzellen, sog. "Biopharmazeutika", werden die entsprechenden Säugerzellen mit DNA stabil transfiziert, die für das jeweilige biologisch aktive Protein (oder seine Untereinheiten) kodiert. Nach dem Transfektionsprozess erhält man normalerweise einen Pool von Millionen unterschiedlich transfizierter Zellen. Daher liegt der entscheidende Schritt zur Herstellung effizienter Produktionszelllinien in der Selektion und Vermehrung von Zellklonen, die zum einen sehr stabil wachsen und zum anderen eine hohe spezifische Produktivität an therapeutischem Protein zeigen (Produktbildung etc.). Da Millionen unterschiedlicher produktexprimierender Zellen vorliegen, ist es entscheidend über hohen Durchsatz und Automatisierung eine Vielzahl von Zellen einzeln analysieren zu können, um geeignete Kandidaten *(Einzelzellklone),* die sowohl sehr robust wachsen als auch hohe Produkttiter liefern, aussortieren zu können. Diesen Vorgang der Einzelzellisolierung und Subkultivierung bezeichnet man auch als - *Klonierung* oder *Reklonierung.*

Die Selektion von transfizierten Zellen ist beispielsweise durch fluoreszenzaktivierte Zellsortierung (FACS) möglich, indem die Expression des therapeutischen Proteins beispielsweise mit der Expression eines Markerproteins verknüpft wird. Hierzu werden beispielsweise fluoreszierende Proteine und dessen Varianten von *Aequorea victoria, Renilla reniformis* oder anderen Spezies, wie zum Beispiel die rot, gelb, violett, grün fluoreszierenden Proteine oder deren Varianten von nichtbiolumineszierenden Organismen wie z.B. *Discosoma sp., Anemonia sp., Clavularia sp., Zoanthus sp.* zusammen mit dem therapeutischen Protein in einer Zelle coexprimiert. Über die Fluoreszenzintensität lassen sich Rückschlüsse auf die spezifische Produktivität und das Wachstumsverhalten der Zellen ziehen.

Es besteht jedoch die Schwierigkeit, typische rekombinante Produktionszellen wie Maus Myeloma- (NSO), Hamster Eierstock- (CHO), oder Hamster Nieren-Zellen (BHK), insbesondere wenn diese an das Wachstum in serumfreien Suspensionskulturen adaptiert sind, also unter modernen produktionsrelevanten Zellkulturbedingungen, einzeln in Kulturgefäße, beispielsweise in Vertiefungen von Mikrotiterplatten, unter serumfreien Kulturbedingungen abzulegen und effektiv zu vermehren (Reklonierung). Werden nur wenige Zellen, beispielsweise weniger als 5 Zellen in ein Kulturgefäß unter serumfreien Bedingungen abgelegt, so lassen sich diese Zellen zumeist gar nicht oder zumindest nicht mehr effizient vermehren. Die Ursache hierfür wird in nicht vorhandenen Zell-Zell-Kontakten, einem größeren Nährstoff-/Wachstumsfaktorbedarf bei geringerer Zelldichte und/oder im Fehlen bzw. zu geringer Konzentration von diffusiblen Signal- und Konditionierungsfaktoren vermutet.

Im Stand der Technik wird das Problem der serumfreien Einzelzellklonierung bei den oben genannten rekombinanten Produktionszellen umgangen, indem Zellklone nach dem Verfahren der "limited dilution" generiert werden. Hierbei werden minimal 5 bis 10 Zellen in serumfreiem Medium in einem Kulturgefäß ausgesät und anschließend durch wiederholte Verdünnungsklonierungen subpassagiert, um statistisch gesehen eine Kultur bestehend aus genetisch identischen Zellen zu erhalten (Verfahren = *limited dilution).* Diese Art der Reklonierung ist zum einen zeitintensiv und zum anderen führt sie zumeist zu genetisch heterogenen Mischkulturen, da das Verfahren auf einer statistischen Berechnung und nicht auf einer tatsächlichen Kultivierung von einzeln abgelegten genetisch identischen Zellen beruht. Diese heterogenen Mischkulturen zeichnen sich in der Regel durch eine eingeschränkte Fermentationsrobustheit und ein heterologes Expressionsprofil aus.

Alternativ lassen sich derzeit Einzelzellklone produktionsrelevanter Zelllinien nur durch Einzelzellablage von serumadaptierten adhärenten Zellen generieren. So erwähnen Meng et al., (2000) beispielsweise ein Verfahren zur Einzelzellablage von adhärent wachsenden CHO Zellen in serumhaltigen Medium. Die von Meng et al., beschriebene Methode bringt aber wesentliche Nachteile mit sich: Aufgrund der Adhärenz der Zellen kann es durch aufwendiges enzymatisches Ablösen der Zellen vom Untergrund (Trypsinbehandlung) zu erheblichen Zellschädigungen und Veränderungen im Wachstumsverhalten und bei der Produktivität der reklonierten Zellen kommen. Ferner müssen die entsprechend gewonnenen Einzelklone anschließend an das serumfreie Wachstum in Suspensionskultur adaptiert werden, was regelmäßig mit hohem Zeitaufwand sowie mit einer veränderten Produktivität der Zelle und Produktqualität verbunden ist (vgl. hierzu u.a. Kaufmann et al., 2001; Mueller et al., 1999).

Durch die Verwendung von Nährzellen, auch feeder-Zellen genannt, bei der Kultivierung adhärent wachsender Zellen können die Wachstumseigenschaften von Zellen positiv beeinflusst bzw. einige Zelltypen überhaupt erst unter Zellkulturbedingen vermehrt werden. Beispiele hierfür sind Mensch-Maus oder Maus-Maus-Hybridomazellen (Hlinak et al 1988, US 5,008,198), primäre Keratinozyten (Rheinwald and Green, 1975; WO9954435), Stammzellen (Williams et al., 1988) und verschiedene Tumorzellen (Wee Eng Lim et al., 2002; Rexroad et al., 1997; Peng et al., 1996; Grigoriev et al., 1996; Sanchez et al., 1991; Butcher et al., 1988; Long et al., 1986; Shneyour et al., 1984; Pintus et al., 1983; Brodin et al., 1983). *Feeder-*Zellen sind zumeist chemisch oder physikalisch in ihrem Wachstum arretierte Zellen, die aufgrund einer speziellen Vorbehandlung ihre Fähigkeit zur Zellteilung eingestellt haben, ansonsten aber noch durchschnittlich für ca. 2 bis 3 Wochen vital sind. Feeder-Zellen sind somit weiterhin in der Lage wachstumsbegünstigende Faktoren in das Medium abzugeben und können somit das anfängliche Wachstum nicht arretierter Zellen begünstigen oder im Fall verschiedener primärer Zellen ermöglichen. Hierzu werden die feeder-Zellen als sog. ,,monolayer" in einem Kulturgefäß ausplattiert. Anschließend werden die zu kultivierenden adhärent wachsenden Zellen auf bzw. zwischen die feeder-Zellen ausplattiert und unter Standardbedingungen kultiviert. Feeder-Zellen lassen sich beispielsweise durch Bestrahlung oder Behandlung mit Mitomycin C (Azirino[2',3':3,4]pyrrolo[1,2-a]indole-4,7-dione,6-amino-8-[[(aminocarbonyl)oxy] methyl] -1,1a,2,8,8a,8b-hexahydro-8a-methoxy-5-methyl-, [1 aR-(1 a.alpha.,8.beta.,8a.alpha., 8b.alpha.)]- (9Cl) (Butcher et al, 1988)) herstellen. Primärzellen wie Milzzellen, Fibroblasten, Blutzellen (Morgan, Darling; Kultur tierischer Zellen. Spektrum Akademischer Verlag 1994, S. 115f) und Makrophagen (Hlinak et al., 1987) sind in Feeder-Zellsystemen beschrieben worden. Im Zusammenhang mit der Produktion von Antikörpern in Hybridomazellen wurde die Verwendung von feeder-Zellen und FACS basierter Zellselektion beschrieben. Hlinak et al. (1987) beispielsweise beschreiben eine Reklonierungseffizienz von 33 bis 57% bei der Reklonierung von adhärent wachsenden Hybridoma-Zellen ausgehend von zwei (2) Zellen pro Kulturgefäß. Bei den verwendeten Hybridoma-Zellen handelte es sich allerdings um adhärent wachsende und an serumhaltiges Medium adaptierte Zellen.

Bei der Verwendung von heterologen, insbesondere von humanen feeder-Zellen für die Generierung von Produktionszellen besteht ein erhebliches Kontaminationsrisiko durch Krankheitserreger, wie beispielsweise Viren, Bakterien oder Mykoplasmen. Ferner benötigen viele der beschriebenen (primären) feeder-Zellen serumhaltiges Medium, was erstens die Kontaminationsgefahr erhöht und zweitens die Gefahr beinhaltet, dass aufwendig an serumfreies Wachstum adaptierte Produktionszellen re-adaptiert werden müssen.

Bei der Verwendung von heterologen feeder-Zellen bedarf es im Allgemeinen einer Gegenselektion der feeder-Zellen. Produktionszellen unterliegen in der Regel einem Selektionsdruck, z.B. durch die Verwendung von Medienzusätzen (Antibiotika wie G418) und/oder nicht komplette Medien (Fehlen von Hypoxanthin, Thymidin). Dieser Selektionsdruck erlaubt die Selektion von Zellen, die die entsprechende genetische Information für ein z.B. rekombinantes Protein aufgenommen und integriert haben. Das so entstandene, an die Produktionszelle angepasste Medium beeinflusst maßgeblich das Wachstum der feeder-Zellen und führt, verbunden mit dem auch ansonsten nicht kompatiblen Produktionsmedium zu einem sehr schnellen Absterben der *feeder-Zellen.* Dadurch ist die Funktion der *feeder-Zelle* nicht mehr gewährleistet.

### Zusammenfassung der Erfindung

Eine Aufgabe der vorliegenden Erfindung war ein effizientes Reklonierungsverfahren zu finden, das, ausgehend von weniger als fünf Zellen, vorzugsweise von einer (1) Einzelzelle, eine Vermehrung produktionsrelevanter Hamsterzellen unter serumfreien Bedingungen und in Suspensionskultur erlaubt. Im Besonderen bestand die Aufgabe darin, entsprechende Verfahren für die Reklonierung von ursprünglich aus Hamster isolierten CHO-, oder BHK-Zellen bereitzustellen.

Diese Aufgaben werden durch die in den Patentansprüchen definierten erfindungsgemäßen Gegenstände gelöst.

Die Erfindung betrifft die Verwendung eines Hamsterzelle als *feeder*-Zellen zur Vermehrung/ Klonierung von in Suspension wachsenden autologen Hamster Zellen unter serumfreien Bedingungen, dadurch gekennzeichnet, dass die als *feeder*-Zelle verwendete Hamsterzelle an serumfreie Kulturbedingungen adaptiert ist und ebenfalls in Suspension gehalten wird, wobei die als *feeder*-Zelle verwendete Hamsterzelle physikalisch oder chemisch inaktiviert wird.

Die erfindungsgemäßen Verfahren zeichnen sich durch eine gute Reklonierungseffizienz von größer 10%, vorzugsweise von größer 20%, insbesondere für einzeln abgelegte Zellen aus. Nach einer weiteren Ausführungsform verfügen die erfindungsgemäßen Reklonierungsverfahren über eine Reklonierungseffizienz von größer 30%, vorzugsweise von größer 40%, besonders bevorzugt von größer 50%, weiter bevorzugt von größer 60%, noch weiter bevorzugt von größer 70%, noch weiter bevorzugt von größer 80%.

Vorliegend gelten Reklonierungseffizienzen von 10 bis größer 65% bei der Reklonierung einzeln abgelegter CHO-Zellen, von 10 bis größer 50% bei der Reklonierung einzeln abgelegter BHK-Zellen als effizient. Werden mehr als eine (1) Zelle pro Kulturgefäß abgelegt, beispielsweise zwei, drei oder vier, so liegt die Reklonierungseffizienz für die jeweilige Zellen über den angegebenen Werten für die Reklonierung von CHO- und BHK- Zellen.

Ein weiterer Aspekt der Erfindung betrifft ein entsprechendes Verfahren zur Reklonierung von Hamster Zellen, bei dem die zu klonierenden Zellen in Gegenwart von 100 bis 200.000 *feeder-*Zellen pro mL Medium vermehrt werden.

Die Expression eines rekombinanten Genprodukts, erfordert zuvor noch die Transfektion der Hamsterzellen mit einer Nukleinsäure, die für das Genprodukt von Interesse kodiert.

Die Hamsterzellen lassen sich hierbei beispielsweise manuell oder durch FACSbasiertes Sortieren vereinzeln und jeweils in ein Zellkulturgefäß mit feeder-Zellen ablegen. Vorzugsweise werden nicht adhärent kultivierte feeder-Zellen verwendet.

Die vorliegende Erfindung betrifft die Verwendung von Hamsterzellen, vorzugsweise CHO- oder BHK-Zellen als *feeder*-Zellen gemäß den Ansprüchen.

Überraschenderweise wurde gefunden, dass durch Einsatz von suspendierten autologen feeder-Zellen, einzelne Hamster- Zellen, in serum- und/oder proteinfreiem Medium abgelegt werden können und zu Kulturen heranwachsen und somit die oben genannten Nachteile bei der Reklonierung, beispielsweise durch *limited dilution,* oder die Adaption monoklonaler Zelllinien an das serumfreie Wachstum in Suspensionskultur überwunden werden können.

Dem gegenüber ist im Stand der Technik (Hügin A.W.: "Cloning of cells with autologous feeder cells" Journal of immunological methods, Elsevier science publishers B.V., Amsterdam, NL, Bd.205, Nr. 2, 14. Juli 1997, Seiten 211-212) lediglich ein Verfahren zur Verbesserung der Reldonierungseffizienz unter Verwendung von autologen Feederzellen in serumhaltigem Medium beschrieben. Die Verwendung einer Hamsterzelle als *feeder*-Zelle zur Vermehrung/ Klonierung von in Suspension wachsenden autologen Hamster Zellen unter serumfreien Bedingungen offenbart Hügin jedoch nicht.

Die erfindungsgemäße Verwendung ermöglicht die Ablage entsprechender Hamsterzellen in serumfreies, proteinfreies oder chemisch definiertes Produktionsmedium, so dass eine üblicherweise durchzuführende Adaption an serumfreies bzw. proteinfreies Produktionsmedium entfällt. Dies führt zu einer wesentlichen Verkürzung von Entwicklungszeiten (ca. 50%) bei der Etablierung von produktionsrelevanten Zelllinien. Ferner führt die Einzelzellablage zu stabilen, homogenen Zellklonen, was für die Produktion von Biopharmazeutika von wesentlicher Bedeutung ist, nicht zuletzt im Hinblick auf regulatorische Erfordernisse bei ihrer Zulassung als Arzneimittel. Darüber hinaus birgt die Verwendung autologer feeder-Zellen zur Reklonierung von produktionsrelevanten CHO-oderBHK- Zellen, z.B. die Verwendung von Hamsterzellen, vorzugsweise von CHO- oder BHK-Zellen, ein wesentlich geringeres Kontaminationsrisiko im Hinblick auf human-pathogene Krankheitserreger als die Verwendung humaner oder weniger gut charakterisierter Zellen.

### Beschreibung der Abbildungen

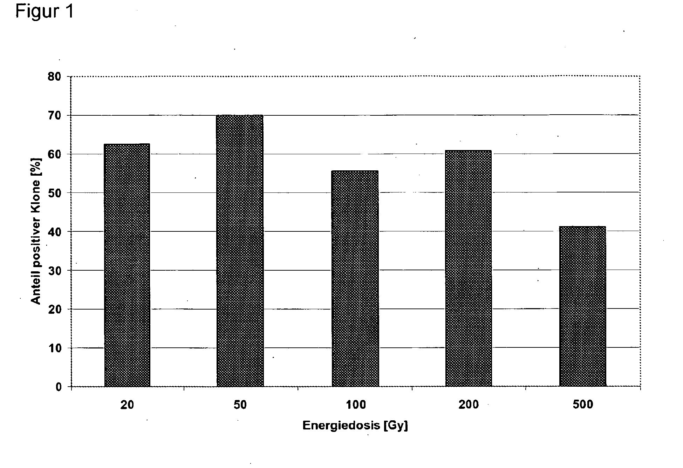

**Abbildung 1** zeigt die Korrelation zwischen Energiedosis, die bei der Herstellung der feeder-Zellen eingesetzt wurde, und Klonierungseffizienz bei der Reklonierung von automatisiert abgelegten CHO-DG44 Einzelzellen. Die Zellablage erfolgte jeweils auf ca. 2000 inaktivierte autologe feeder-Zellen. Aus den Grafiken ist ersichtlich, dass mit der Energiedosis von 20-500 Gy bestrahlte feeder-Zellen noch ausreichend Faktoren in das Medium abgeben, so dass mehr als 65% der abgelegten Einzelklone bei 50 Gy zu Kolonien heranwachsen.

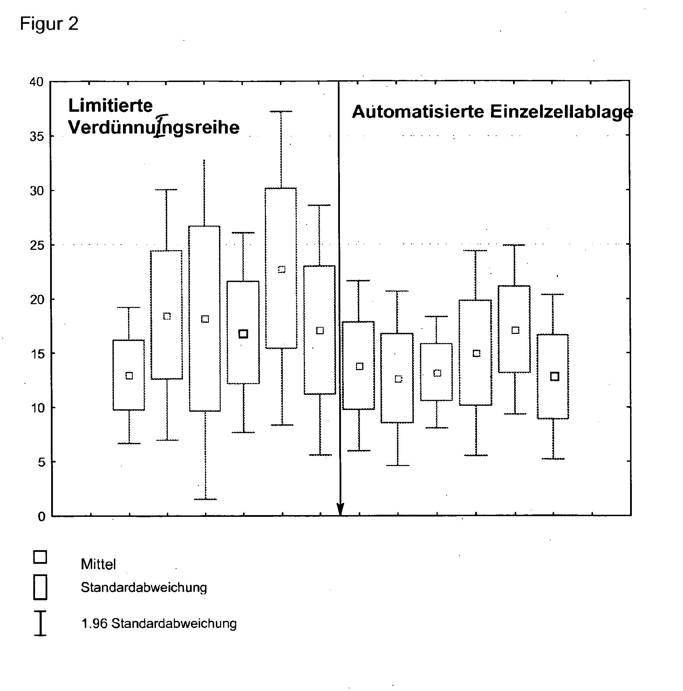

**Abbildung 2** zeigt die Produktivität von Antikörper-exprimierenden CHO-DG44 Kulturen, die durch limitierte Verdünnung (linke Spalte) oder Einzelzellablage (rechte Spalte) erhalten wurden. In der linken Spalte ist die Produktivität von jeweils 6 Kulturen dargestellt, die mittels limitierter Verdünnung erhalten wurden. In der rechten Hälfte ist die Produktivität von 6 Einzelklonen aus der automatisierten Einzelzellablage dargestellt. Zur Produktivitätsbestimmung wurden drei Parallelversuche pro Zellklon/Kultur durchgeführt. Im Gegensatz zur Klonierung mittels ,,limitierter Verdünnung" führte die Klonierung durch automatisierte Einzelklonablage zu einer wesentlich geringeren Streuung in der Produktivität. Die parallel kultivierten und aus einem Einzelklon abgeleiteten Subkulturen zeigen eine wesentlich höhere Homogenität im Hinblick auf ihre Produktivität im Vergleich zu den nach limitierter Verdünnung gewonnenen Subkulturen.

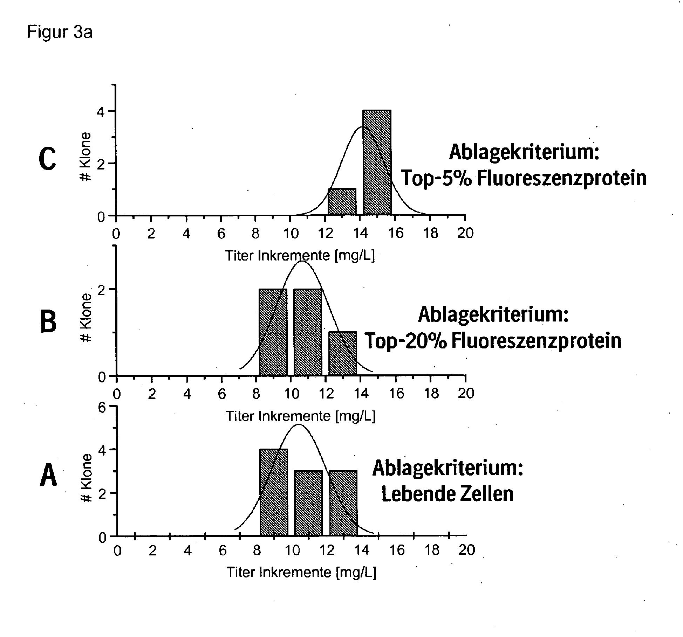

**Abbildung 3a** zeigt den Produkttiter von nach unterschiedlichen Kriterien sortierten und einzeln abgelegten CHO-DG44 Zellklonen. In der untersten Grafik A wurden Einzelzellen abgelegt, die lediglich dem Sortierungskriterium ,,lebende Zelle" genügen. Dieses Kriterium wurde über die Forward-Side-Scatter-Auftragung im Flow Cytometer definiert. Eine Auswahl nach Fluoreszenz der Zellen erfolgte nicht. Dagegen wurde in der mittleren und oberen Grafik B und C zusätzlich zum Sortierungskriterium "lebende Zelle" das Sortierungskriterium "Fluoreszenz der Zellen" logisch verknüpft. Eine nähere Spezifikation des Sortierungskriteriums "Fluoreszenz der Zellen" erfolgte weiterhin über die Fluoreszenzintensität. Hierzu wurden zum einen die Top-20% fluoreszierenden und zum anderen die Top-5% fluoreszierenden Zellklone einzeln abgelegt. An der Rechtsverschiebung des Histogramms C ist zu erkennen, dass der Anteil der hochexprimierenden Zellklone bei Verwendung des Kriteriums Top-5% fluoreszierende Zellen gegenüber den übrigen angewandten Sortierungskriterien bei A und B deutlich zunimmt.

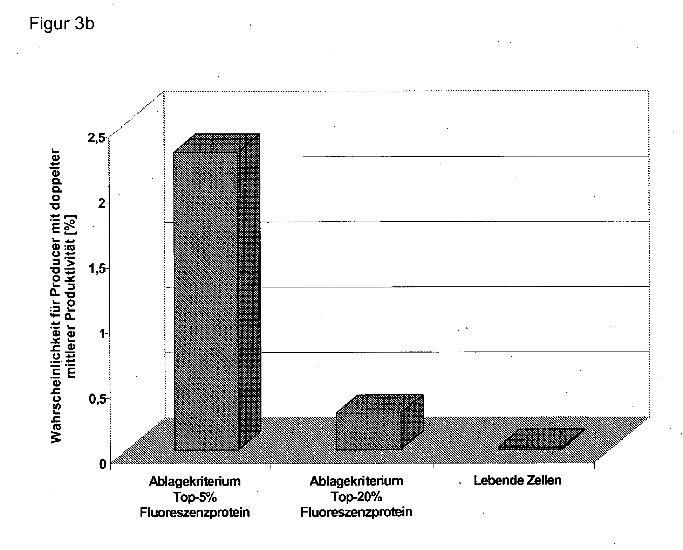

**Abbildung 3b** basiert auf den in Abbildung 3a gezeigten Daten. Dargestellt ist die prozentuale Wahrscheinlichkeit, einen Produzent mit doppelter mittlerer Produktivität (=Hochproduzent) zu erhalten. An die Daten, die für alle lebenden Zellen erhalten wurden, wurde eine Normalverteilung angepasst und der mittlere Titer bestimmt. Aus der Verteilungsfunktion für die Normalverteilung wurde dann der Prozentsatz errechnet, bei welchem die über die Einzelzellablage erhaltene Zellklone den doppelten oder höheren Titer besitzen. Diese Prozentzahl wurde in Abbildung 3b aufgetragen. Aus der Grafik ist ersichtlich, dass die Wahrscheinlichkeit für einen Hochproduzenten bei zusätzlicher Verwendung des Kriteriums Top-5% im Vergleich zur alleinigen Verwendung des Kriteriums ,,lebende Zellen" um mehr als das 20-fache zunimmt.

### Detaillierte Beschreibung der Erfindung und bevorzugte Ausführungsformen

Bevor die Erfindung nachfolgend anhand nicht-beschränkender Ausführungsbeispiele näher erläutert wird, sei darauf verwiesen, dass die Verwendung unbestimmter Artikel, beispielsweise "ein" oder "eine", sowie die bestimmter Artikel, wie ,,der", ,,die", ,,das" Einzahl u n d Mehrzahl des entsprechenden Begriffs einschließen, es sei denn, dass eine der beiden Formen explizit ausgeschlossen und auf eine bestimmte Form (Einzahl, Mehrzahl) verwiesen wird. So schließt der Begriff "eine Zelle" automatisch "mehrere Zellen" mit ein, es sei denn es wird explizit darauf verwiesen, dass nur eine einzige Zelle gemeint ist. Einzahl ist beispielsweise dort explizit gemeint, wo "ein" durch (1) ergänzt ist.

### Definitionen

Der Begriff "Klonierung/Reklonierung", ,,klonieren/reklonieren" meint im Zusammenhang mit Zellkultur eine Technik, mit deren Hilfe man eine Zellpopulation identischer Zellen aus einer Ursprungszelle erhalten kann. Der Ausdruck ,,Zellklonierung" oder auch ,,Einzelzellklonierung" meint somit ein Verfahren, bei dem aus einem Zellpool mit Zellen unterschiedlichen Genotyps einzelne Zellen identifiziert, isoliert und anschließend zu einer Zellpopulation bestehend aus einer Vielzahl genetisch identischer Zellen vermehrt werden. Werden die Zellen hierzu einzeln abgelegt, d.h. lediglich eine (1) Zelle pro Kulturgefäß, und anschließend zu einer Zellpopulation identischer Zellen expandiert, so handelt es sich bei dem Verfahren um eine "direkte Einzelzellklonierung". Werden mehrere Zellen gleichzeitig in ein Kulturgefäß abgelegt, zu einer Zellpopulation expandiert und diese durch wiederholtes Verdünnen *(= limited dilution)* in Zellpopulationen identischer Zellen aufgeteilt, so beschreibt dies ein Verfahren der "indirekten Klonierung".

Bei "Einzelklonen" handelt es sich um genetisch identische Zellen, die von einer (1) einzigen Zelle abstammen. Eine Zellpopulation bestehend aus identischen Zellen gleicher Abstammung bezeichnet man folglich auch als "monoklonale Zellpopulation". Kommt es während der Kultivierung von Zellen gleichen Ursprungs zu spontanen Genomveränderungen, beispielsweise zu Mutationen und/oder Translokationen, so werden die einzelnen Zellen dieser Zellpopulation dennoch als identische Zellen im Sinne der vorliegenden Erfindung angesehen, und die Kultur als monoklonale Zellpopulation verstanden. Dagegen handelt es sich bei einem Pool an stabil transfizierten Zellen (Transfektanten) nicht um Zellklone gleicher Abstammung, also nicht um eine monoklonale Zellpopulation, auch wenn genetisch identische Ausgangszellen mit einer identischen Nukleinsäure transfiziert werden.

Der Begriff ,,Subklone/Subkulturen" bezieht sich auf verschiedene Zellgenerationen, die ausgehend von einer Ursprungszelle/Ursprungskultur durch einfaches oder mehrfaches Passagieren der sich teilenden Zellen entstehen. Von ,,Subklonen/ Subkulturen" spricht man beispielsweise, wenn identische Zellen/Zellkulturen über mehrere Generationen kultiviert und vermehrt werden.

Unter einer ,,effektiven oder effizienten Reklonierung" versteht man eine Klonierungseffizienz von zumindest 10%, vorzugsweise von zumindest 20%, weiter bevorzugt von zumindest 30% und noch weiter bevorzugt von zumindest 40%. Nach einer besonders bevorzugten Ausführungsform der vorliegenden Erfindung versteht man unter einer effektiven Reklonierung eine Klonierung mit einer Effizienz von mindestens 50%, vorzugsweise von mindestens 60%, besonders bevorzugt von mindestens 70% und noch weiter bevorzugt von mindestens 80%

Der Begriff ,,Klonierungseffizienz" ist definiert als der Prozentsatz an Zellen, die nach der Ablage vitale Zellpopulationen von vorzugsweise mehr als 50 Zellen bilden können. Werden beispielsweise bei einer Zellsortierung 50 Zellen auf 50 Kulturgefäße verteilt und wachsen 25 dieser 50 einzeln abgelegten Zellen zu Kulturen heran, so beträgt die Klonierungseffizienz 50% (25 von 50).

Der Ausdruck ,,teilungsfähig/expandierbar" im Sinne der vorliegenden Erfindung beschreibt das Potential einer Zelle/Zellpopulation sich endlos, zumindest aber über 2, vorzugsweise 4, Passagen teilen zu können. Dieses Potential kann beispielsweise durch Bestrahlung mit ^{[137]}Cs- oder durch Mitomycin C Behandlung reduziert oder gänzlich gestört werden.

Der Ausdruck "Derivat/Abkömmling" bezieht sich auf Zellen, die genetisch auf eine bestimmte Ausgangszelle zurückzuführen sind und beispielsweise durch Subkultivierung (mit oder ohne Selektionsdruck) entstehen und/oder durch genetische Manipulation generiert werden. Re-Isolierungen von Zellen des gleichen Zelltyps sind ebenfalls von dem Ausdruck ,,Derivat/Abkömmling" erfasst. So stellen beispielsweise alle CHO-Zelllinien Derivate/Abkömmlinge der von Puck et al., 1958 isolierten Hamsterovarzellen aus *Cricetulus griseus* dar, unabhängig ob sie durch Subkultivierung, Re-Isolierung oder genetische Manipulationen erhalten wurden.

Der Begriff "autologe *feeder-Zelle"* meint, dass *feeder-Zelle* und die zu kultivierende Zelle der gleichen Art entstammen, beispielsweise *Cricetulus griseus* oder *Mesocricetus auratus.* Nach einer besonders bevorzugten Ausführungsform liegt eine ,,autologe *feeder-Zelle"* vor, sofern *feeder-Zelle* und die zu kultivierende Zelle der gleichen Art entstammen und über den gleichen Gewebstropismus verfügen (z.B. Ovarzellen aus *Cricetulus griseus -* CHO-Zellen). Nach einer besonders bevorzugten Ausführungsform handelt es sich bei einer *feeder-Zelle* um eine autologe *feeder-Zelle,* wenn sowohl *feeder-*Zelle als auch zu kultivierende Zelle der gleichen Basiszelle entstammen, beispielsweise wenn es sich bei beiden Zellen ursprünglich um CHO-DG44 Zellen bzw. Abkömmlinge dieser Zelle handelt. Nach einer weiter bevorzugten Ausführungsform vermittelt die *feeder-Zelle* die selben Resistenzen, z.B. gegenüber Antibiotika, wie die zu kultivierende Zelle. Dies ist insbesondere von Vorteil, wenn die Zellablage in Gegenwart eines Selektionsmittels, z.B. eines Antibiotikums, erfolgt.

Der Begriff ,,serumfrei" meint Kulturmedien wie auch Kultivierungsbedingungen, die dadurch gekennzeichnet sind, dass Zellen in Abwesenheit von tierischem und/oder humanem Serum, vorzugsweise in Abwesenheit von jeglichen aus Serum isolierten Proteinen, vorzugsweise in Abwesenheit von nicht rekombinant gewonnenen Proteinen kultiviert werden. Folglich meint der Ausdruck "an serumfreie Bedingungen adaptierte Zellen" solche Zellen, die in Abwesenheit von tierischem oder humanem Serum bzw. Serumproteinen vermehrt werden können.

Der Begriff ,,proteinfrei" meint, dass das Kulturmedium keine tierischen Proteine enthält, wobei aus Bakterien, Hefen oder Pilzen isolierte Proteine nicht als tierische Proteine verstanden werden.

Der Begriff "chemisch definiert" beschreibt ein Zellkulturmedium, welches serumfrei, vorzugsweise auch proteinfrei ist, und das aus chemisch definierten Substanzen besteht. Chemisch definierte Medien bestehen somit aus einer Mischung aus vorwiegend reinen Einzelsubstanzen. Ein Beispiel für ein chemisch definiertes Medien ist beispielsweise das CD-CHO-Medium der Firma Invitrogen (Carlsbad, CA, US).

Der Ausdruck "eine in Suspension kultivierbare Zelle" bezieht sich auf Zellen, die an das Wachstum in flüssigen Kulturen ("Suspensionskulturen") adaptiert sind und deren Fähigkeit zu Adhäsion an Gefäßoberflächen, beispielsweise an Zellkulturschalen / -flaschen eingeschränkt bzw. verloren gegangen ist. Zellen, die sowohl an serumfreies Wachstum als auch an das Wachstum in Suspension adaptiert sind, werden als "an serumfreies Medium adaptierte und nicht adhärente Zellen" bezeichnet. Stellt man aus solchen Kulturen *feeder-Zellen* her, so handelt es sich definitionsgemäß bei diesen Zellen um ,,an serumfreies Medium adaptierte und nicht adhärente feeder-Zellen".

### Beschreibung der Erfindung

Die vorliegende Erfindung betrifft die Verwendung einer Hamsterzelle als *feeder*-Zelle zur Vermehrung/ Klonierung von in Suspension wachsenden autologen Hamster Zellen unter serumfreien Bedingungen, dadurch gekennzeichnet, dass die als *feeder*-Zelle verwendete Hamsterzelle an serumfreie Kulturbedingungen adaptiert ist und ebenfalls in Suspension gehalten wird, wobei die als *feeden*-Zelle verwendete Hamsterzelle physikalisch oder chemisch inaktiviert wird. Bevorzugt werden weniger als fünf, beispielsweise vier, drei, zwei, oder eine (1) Hamsterzelle(n) in Gegenwart von autologen Hamster-feeder-Zellen, in einem Kulturgefäß unter serumfreien Bedingungen abgelegt und unter serumfreien Bedingungen kultiviert und vermehrt. Bei einer weiter bevorzugten Ausführungsform wird eine (1) einzelne Hamsterzelle in Gegenwart von autologen Hamster-feeder-Zellen in ein Kulturgefäß unter serumfreien Bedingungen abgelegt, unter serumfreien Bedingungen kultiviert und vermehrt. In einer weiter bevorzugten Ausführungsform handelt es sich bei der abgelegten und zu kultivierenden Zelle jeweils um eine in Suspensionskultur wachsende Zelle.

Wird lediglich eine (1) Zelle pro Kulturgefäß abgelegt und zu einer Zellpopulation vermehrt, so handelt es sich bei jeder einzelnen heranwachsenden Zellpopulation um eine monoklonale Zellpopulation und bei dem Verfahren um ein Verfahren zur direkten Einzelzellklonierung. Werden mehr als eine (1) einzelne Zelle pro Kulturgefäß abgelegt und vermehrt, beispielsweise zwei, drei oder vier Zellen, so handelt es sich bei den heranwachsenden Zellpopulationen um sog. Mischklone. Diese können anschließend durch direkte Einzelzellklonierung oder auch nach herkömmlichen Verfahren, beispielsweise durch wiederholtes Ausverdünnen der Zellpopulationen (= *limited dilution)* in sog. statistische monoklonale Zellpopulationen überführt werden (siehe beispielsweise Morgan, Kultur tierischer Zellen, Seiten 113 und 114, Spektrum Akademischer Verlag 1994).

Mit der durch die vorliegende Erfindung bereitgestellten Verwendung lassen sich insbesondere Säugerzellen der Unterfamilie *Cricetinae,* beispielsweise der Gattungen *Cricetulus* oder *Mesocricetus,* sowie aus diesen isolierte Zelllinien einschließlich ihrer Abkömmlinge/Derivate, vermehren und auch klonieren. Vorzugsweise eignet sich die erfindungsgemäße Verwendung zur Vermehrung/Klonierung von Hamsterzellen, sowie von hiervon abgeleiteten stabilen Zelllinien.

Gemäß einer besonders bevorzugten Ausführungsform betrifft die erfindungsgemäße Verwendung Hamsterzellen der Gattung *Cricetulus* (Chinesischer Zwerghamster) sowie von aus dieser Gattung isolierten oder von den isolierten Zellen abgeleitete stabile Zelllinien, beispielsweise von CHO, CHO-K1, CHO-DUKX, CHO-DUKX B1 oder CHO-DG44 Zellen sowie von Derivaten/Abkömmlingen dieser Zelllinien. Besonders erfindungsgemäß ist eine Verwendung, bei der CHO-DG44, CHO-DUKX, und CHO-K1, insbesondere CHO-DG44 und CHO-DUKX Zellen in Gegenwart autologer *feeder-*Zellen vermehrt und kloniert werden. Mit Hilfe der erfindungsgemäßen Verwendung lassen sich auch Zellen aus *Mesocricetus auratus* (Syrischer Goldhamster) sowie hieraus isolierte oder hiervon abgeleitete stabile Zelllinien, beispielsweise BHK21 oder BHK TK- Zellen sowie Derivate/Abkömmlinge dieser Zelllinien vermehren und auch klonieren. Vorzugsweise werden weniger als fünf, beispielsweise vier, drei, zwei, oder vorzugsweise lediglich eine (1) Zelle(n) in Gegenwart von autologen feeder-Zellen in ein Kulturgefäß unter serumfreien Bedingungen abgelegt und unter serumfreien Bedingungen kultiviert und vermehrt.

Weitere Beispiele für Hamsterzellen, die erfindungsgemäß vermehrt und kloniert werden können, sind in der folgenden Tabelle 1 angegeben.

**Tabelle 1: HamsterZelllinien**

| Zelllinie | Hinterlegungsnummer |
|---|---|
| BHK21 | ATCC CCL-10 |
| BHK TK- | ECACC No. 85011423 |
| HaK | ATCC CCL-15 |
| 2254-62.2 (BHK-21-Derivat) | ATCC CRL-8544 |
| CHO | ECACC No. 8505302 |
| CHO-K1 | ATCC CCL-61 |
| CHO-DUKX (= CHO duk-, CHO/dhf() | ATCC CRL-9096 |
| CHO-DUKX B1 | ATCC CRL-9010 |
| CHO-DG44 | Urlaub et al., Cell 33[2], 405-412, 1983 |
| CHO Pro-5 | ATCC CRL-1781 |
| V79 | ATCC CCC-93 |
| B14AF28-G3 | ATCC CCL-14 |
| CHL | ECACC No. 87111906 |

Erfindungsgemäß werden die entsprechenden Hamsterzellen vorzugsweise unter serumfreien Bedingungen etabliert, kultiviert und abgelegt. Gegebenenfalls erfolgen diese Schritte in Medien, die frei von tierischen Proteinen/Peptiden und/oder chemisch definiert sind. Beispiele für im Handel erhältliche Medien sind Ham's F12 (Sigma, Deisenhofen, DE), RPMI-1640 (Sigma), Dulbecco's Modified Eagle's Medium (DMEM; Sigma), Minimal Essential Medium (MEM; Sigma), Iscove's Modified Dulbecco's Medium (IMDM; Sigma), CD-CHO (Invitrogen, Carlsbad, Ca., USA), CHO-S-SFM-11 (Invitrogen), serumfreies CHO-Medium (Sigma) und proteinfreies CHO-Medium (Sigma). Jedes dieser Medien kann gegebenenfalls mit verschiedenen Verbindungen ergänzt werden, z.B. Hormonen und/oder anderen Wachstumsfaktoren (z.B. Insulin, Transferrin, epidermalem Wachstumsfaktor, Insulin-ähnlichem Wachstumsfaktor), Salzen (z.B. Natriumchlorid, Calcium, Magnesium, Phosphat), Puffern (z.B. HEPES), Nukleosiden (z.B. Adenosin, Thymidin), Glutamin, Glukose oder anderen äquivalenten Nährstoffen, Antibiotika und/oder Spurenelementen. Zur Selektion von genetisch modifizierten Zellen, die ein oder mehrere Selektionsmarkergene exprimieren, kann dem Medium ein oder mehrere geeignete Selektionsmittel, z.B. Antibiotika, zugefügt werden.

Bisher ist die Verwendung von feeder-Zellen zur Kultivierung von Zellen im Zusammenhang mit der Kultivierung adhärent wachsender Zellen beschrieben (z.B. Wee Eng Lim et al., 2002; Rexroad et al., 1997; Peng et al., 1996; Grigoriev et al., 1996; Sanchez et al., 1991; Butcher et al., 1988; Long et al., 1986; Shneyour et al., 1984; Pintus et al., 1983; Brodin et al., 1983. Hierbei werden adhärente feeder-Zellen in ein Kulturgefäß bzw. auf einen Träger als Einzelzellschicht (engl. *monolayer)* ausgelegt und die zu kultivierenden Zellen auf diesem *monolayer* kultiviert. Da die feeder-Zellen ihre Fähigkeit zum weiteren Wachstum verloren haben (entweder natürlicherweise oder durch künstliches Einwirken) können sich die zu kultivierenden Zellen vermehren ohne von den feeder-Zellen überwachsen zu werden. Hierbei sind sowohl feeder- als auch die zu kultivierenden Zellen adhärent wachsende Zellen.

Dem gegenüber ermöglicht die vorliegende Erfindung gemäß einer weiteren erfindungsgemäßen Ausführungsform die Vermehrung von in Suspension wachsenden Hamster Zellen, entweder unter Verwendung adhärenter autologer feeder-Zellen oder nach einer weiter bevorzugten Ausführungsform unter Verwendung von ebenfalls in Suspension gehaltenen autologen Hamster- *feeder-Zellen.* Die Verwendung von in Suspension gehaltenen autologen Hamster *feeder-Zellen* ist insbesondere bevorzugt, wenn sowohl die *feeder-Zelle(n)* als auch die zu kultivierende Zelle(n) der gleichen Basiszelle entstammen, beispielsweise wenn es sich bei beiden Zellen ursprünglich um Zellen handelt, die an das Wachstum in Suspension adaptiert worden sind.

Die Anzahl der zu verwendeten autologen Hamster-*feeder-Zellen* bei der Vermehrung/Klonierung von den hier beschriebenen Hamsterzellen hängt grundsätzlich von der Natur der zu vermehrenden und zu klonierenden Hamsterzelle ab und lässt sich durch einfache Titrationsversuche für jeden Zelltyp bestimmen. Die erfindungsgemäßen Verfahren zur Vermehrung/Klonierung von Hamsterzellen werden beispielsweise in Gegenwart von zumindest mehr als 100 autologen *feeder-Zellen* pro mL Medium durchgeführt, vorzugsweise in Gegenwart von 100 bis 200.000 autologen *feeder-Zellen* pro mL Medium. In einer weiter bevorzugten Ausführungsform erfolgt die Vermehrung/Klonierung der Hamsterzellen in Gegenwart von 500 bis 50.000 autologen *feeder-Zellen* pro mL Medium. Noch weiter bevorzugt ist ein Verfahren, bei dem die Vermehrung/Klonierung der Hamsterzellen in Gegenwart von 500 bis 10.000 autologen *feeder-Zellen* pro mL Medium, vorzugsweise in Gegenwart von 2.000 bis 10.000 autologer *feeder-Zellen* pro mL Medium durchgeführt wird.

Entsprechend der Definition des Begriffs "autologe *feeder-Zelle"* betrifft die vorliegende Erfindung Hamsterzellen, vorzugsweise der Unterfamilie *Cricetinae,* weiter bevorzugt der Gattungen *Cricetulus* oder *Mesocricetus,* die als *feeder-*Zellen verwendet werden, wenn es sich bei den abgelegten und zu vermehrenden/klonierenden Säugerzellen um CHO- oder BHK-Zellen handelt . Bevorzugt werden CHO-Zellen als *feeder-Zellen* verwendet, wenn es sich bei den abgelegten und zu vermehrenden/klonierenden Säugerzellen um CHO-Zellen handelt. Weiterhin werden bevorzugt BHK-Zellen als *feeder-Zellen* verwendet, wenn es sich bei den abgelegten und zu vermehrenden/klonierenden Säugerzellen um BHK-Zellen handelt.

Für den Fall, dass die abgelegten und zu kultivierenden Hamsterzellen in Gegenwart eines Selektionsmittels kultiviert werden, eignet sich die Verwendung von autologen *feeder-Zellen,* die ebenfalls über das Resistenz vermittelnde Selektionsmarkergen verfügen, da somit ein schnelleres Absterben der feeder-Zellen in Gegenwart des Selektionsmittels verhindert werden kann. Demnach betrifft die vorliegende Erfindung insbesondere die Verwendung vonHamster Zellen, als feeder-Zellen dadurch gekennzeichnet, dass weniger als fünf, beispielsweise vier, drei, zwei, oder eine (1) dieser Hamsterzelle(n) in Gegenwart von autologen *feeder-Zellen,* in einem Kulturgefäß unter serumfreien Bedingungen abgelegt und unter serumfreien Bedingungen kultiviert und vermehrt werden (wird), wobei die autologen *feeder-Zellen* und die abgelegten Hamsterzelle(n) jeweils über zumindest ein Selektionsmarkergen verfügen, welches eine Resistenz gegenüber einem Selektionsmittel vermittelt, und zumindest die Vermehrung der zu klonierenden Hamsterzelle(n) unter serumfreien Bedingungen in Gegenwart des besagten Selektionsmittels erfolgt, gegenüber dem sowohl die *feeder-Zelle* als auch die zu klonierende Hamsterzelle resistent ist.

Die autologen *feeder-Zellen* lassen sich beispielsweise durch Bestrahlung mit einer radioaktiven Strahlenquelle herstellen, zum Beispiel durch Bestrahlung mit dem Cäsium-Isotop 137 (¹³⁷CS). Als vorteilhaft für die hier beschriebenen Verfahren zur Vermehrung/Klonierung von Säugerzellen in Gegenwart autologer *feeder-Zellen* ist die Bestrahlung mit einer Energiedosis zwischen 1 und 1.000 Gy. Besonders vorteilhaft ist hierbei die Verwendung einer Energiedosis zwischen 10 und 500 Gy, weiter vorteilhaft zwischen 20 und 200 Gy. Im Zusammenhang mit der Klonierung von CHO-Zellen hat sich gezeigt, dass die Verwendung von autologen *feeder-Zellen,* vorzugsweise CHO-Zellen, vorteilhaft ist und zu einer effizienten Klonierungseffizienz führt, die mit einer Energiedosis zwischen 1 und 500 Gy bestrahlt wurden. Besonders vorteilhaft erwies sich hierbei die Verwendung autologer *feeder-Zellen* die mit einer Energiedosis zwischen 20 und 100 Gy, vorzugsweise mit etwa 50 Gy bestrahlt wurden. Grundsätzlich lässt sich die optimale Energiedosis für jede Zelle experimentell ermitteln, indem *feeder-Zellen* mit unterschiedlichen Gesamtstrahlendosen behandelt werden, und analog zu der in den Ausführungsbeispielen beschriebenen Methode jeweils die Klonierungseffizienz in Abhängigkeit zur Strahlendosis bestimmt wird. Neben der Gammabestrahlung mit ¹³⁷Cs und ⁶⁰Co (Kobalt-Isotop 60) eignet sich beispielsweise auch eine Behandlung mit UV-Strahlung, Elektronenstrahlung, radioaktiver Strahlung, Neutronenstrahlung und Mikrowellenstrahlung.

Die *feeder-Zellen* lassen sich direkt oder nach Kryokonservierung, beispielsweise in flüssigen Stickstoff, bei einem der erfindungsgemäßen Verfahren zur Vermehrung/Klonierung von Hamsterzellen einsetzten. Verfahren zur Kryokonservierung von Säugerzellen sind den Fachmann bekannt und beispielhaft in Freshney (Hers.), Animal Cell culture - a practical approach, IRL-Press 1986, Seiten 73 - 78 beschrieben, auf die hier inhaltlich Bezug genommen wird.

Die vorliegenden Verwendung eignet sich dazu, die abgelegten Hamsterzellen bis zu einer Dichte von 1x 10⁵ bis 4 x 10⁶ / mL Medium in dem Kulturgefäß zu vermehren, in dem sie ursprünglich abgelegt wurden. Vorzugsweise erfolgt eine erste Passagierung bei einer Zelldichte von 2 x 10⁵ bis 8 x 10⁵ / mL Medium, insbesondere bei einer Zelldichte von 2 × 10⁵ bis 5x 10⁵ / mL Medium.

Die erfindungsgemäße Verwendung von Hamsterzellen als feeder-Zellen zur Vermehrung/Klonierung der hier beschriebenen Hamsterzellen bewirkt eine effiziente Reklonierungseffizienz von bevorzugt zumindest 10%, vorzugsweise zumindest 20%, weiter bevorzugt zumindest 30% und noch weiter bevorzugt zumindest 40% beträgt. Nach einer besonders bevorzugten Ausführungsform beträgt die Reklonierungseffizienz mindestens 50%, vorzugsweise mindestens 60% und besonders bevorzugt mindestens 70% und noch weiter bevorzugt zumindest 80% .

Gemäß den hier beschriebenen Beispielen wurde für CHO-Zellen eine Reklonierungseffizienz einschließlich größer 65% erzielt.

Bevorzugt enthält die jeweilige reklonierte/versuchte Zelle ein oder mehrere Gene von Interesse , das/die für ein oder mehrere herzustellende Produkte (Polypeptiden, Proteinen, Nukleinsäuren, etc.), kodiert. Bevorzugt handelt es sich um rekombinante Produkte. Vorzugsweise handelt es sich bei der , BHK- entsprechenden Zelle um eine CHO-oder BHK- Zelle sowie um Derivate/Abkömmlinge dieser Zelllinien. Es kann sich hierbei allerdings auch um jede andere Hamster-Zelle, z.B. um eine der in Tabelle 1 aufgelisteten Zellen, handeln.

Bei dem/den zu produzierenden Gen(en) von Interesse kann es sich um natürlicherweise in der Wirtszelle vorhandene Gene handeln oder aber auch um künstlich in die Zellen eingebrachte Gene. Per Definition wird jede Sequenz oder jedes Gen, das in eine Zelle eingebracht wird, in Bezug auf diese Zelle als "heterologe Sequenz" oder "heterologes Gen" bezeichnet, selbst dann, wenn die einzubringende Sequenz oder das einzubringende Gen identisch zu einer endogenen Sequenz oder einem endogenen Gen der Zelle ist. Beispielsweise ist ein Hamster-Aktingen, das in eine Hamster-Zelle eingebracht wird, per Definition ein heterologes Gen. Kodiert dieses heterologe Gen für ein Gen von Interesse, so spricht man auch von einem ,,heterologen Gen von Interesse".

Ein heterologes Gen von Interesse kann auf verschiedene Weisen in die Zelle eingebracht werden, beispielsweise durch virale Transformation, Transfektion oder aber auch durch Mikroinjektion. Dabei kann das heterologe Gen von Interesse als lineare DNA in die Zelle eingebracht werden oder aber als Teil eines Expressionsvektors. Es sind eine Vielzahl von eukaryontischen Expressionsvektoren bekannt, die multiple Klonierungsstellen zur Einführung von einem oder mehreren heterologen Genen sowie deren Expression ermöglichen. Kommerzielle Anbieter sind unter anderem Firmen wie Stratagene, La Jolla, CA, USA; Invitrogen, Carlsbad, CA, USA; Promega, Madison, WI, USA oder BD Biosciences Clontech, Palo Alto, CA, USA. Die Transfektion der Zellen mit einer DNA oder einem Expressionsvektor, die/der für ein oder mehrere Gene von Interesse kodiert/kodieren, erfolgt nach üblichen Methoden, wie beispielhaft in Sambrook et al., 1989 oder Ausubel et al., 1994 beschrieben. Geeignete Transfektionsmethoden sind z.B. die Liposomenvermittelte Transfektion, Calciumphosphat-Copräzipitation, Elektroporation, Polykationen (z.B. DEAE-Dextran)-vermittelte Transfektion, Protoplastenfusion, Mikroinjektion und virale Infektionen. Vorzugsweise wird eine stabile Transfektion durchgeführt, wobei die DNA-Moleküle entweder in das Genom der Wirtszelle oder ein artifizielles Chromosom/Minichromosom integriert werden oder in stabiler Weise episomal in der Wirtszelle enthalten sind. Die Transfektionsmethode, die die optimale Transfektionsfrequenz und Expression von einem oder mehreren heterologen Genen von Interesse in der jeweiligen Zelle ermöglicht, ist dabei bevorzugt.

Das heterologe Gen von Interesse ist zumeist funktionell mit einen Promotor, der die Transkription des Gens von Interesse ermöglicht, sowie mit weiteren regulatorischen Elementen verknüpft, die eine Transkription und Translation (Expression) des Gens von Interesse ermöglichen oder in ihrer Effizienz steigern.

Als "Promotor" wird eine Polynukleotidsequenz bezeichnet, die die Transkription der mit ihr funktionell verknüpften Gene oder Sequenzen ermöglicht und kontrolliert. Ein Promotor enthält Erkennungssequenzen für die Bindung der RNA-Polymerase und die Initiationsstelle der Transkription (Transkriptionsinitiationsstelle). Zur Expression einer gewünschten Sequenz in einem bestimmten Zelltyp oder einer Wirtszelle muss jeweils ein geeigneter, funktionaler Promotor gewählt werden. Der Fachmann kennt eine Vielzahl von Promotoren aus verschiedenen Quellen, einschließlich konstitutiver, induzierbarer und reprimierbarer Promotoren. Sie sind in Datenbanken, z.B. GenBank, hinterlegt und können als eigenständige oder innerhalb von Polynukleotidsequenzen klonierte Elemente von kommerziellen oder individuellen Quellen bezogen werden. In induzierbaren Promotoren kann die Aktivität des Promotors in Reaktion auf ein Signal reduziert oder verstärkt werden. Ein Beispiel für einen induzierbaren Promotor stellt der Tetracyclin (tet)-Promotor dar. Dieser enthält Tetracyclin-Operatorsequenzen (tet0), die durch ein Tetracyclin-reguliertes Transaktivatorprotein (tTA) induziert werden können. In der Anwesenheit von Tetracyclin wird die Bindung von tTA an tetO inhibiert. Beispiele für weitere induzierbare Promotoren sind der jun-, fos-, Metallothionin- und Hitzeschockpromotor (siehe auch Sambrook et al., 1989; Gossen et al., 1994). Unter den Promotoren, die für eine hohe Expression in Eukaryonten besonders gut geeignet sind, befinden sich der Hamster-Ubiquitin/S27a-Promotor (WO 97/15664), der SV40 early Promotor, der Adenovirus major late Promotor, der Maus Metallothionin-1 Promotor, die lange terminale Repeatregion des Rous Sarcoma Virus und der early Promotor des humanen Cytomegalie-Virus. Beispiele für andere heterologe Säugerpromotoren sind der/die Aktin-, Immunglobulin-, oder Hitzeschockpromotor(en).

Beispielsweise kann der Promotor mit Enhancer-Sequenzen funktionell verknüpft werden, um die Transkriptionsaktivität zu steigern. Hierzu können ein oder mehrere Enhancer und/oder mehrere Kopien einer Enhancer-Sequenz verwendet werden, beispielsweise ein CMV- oder SV40-Enhancer.

Mit dem Ausdruck "Enhancer" wird eine Polynukleotidsequenz bezeichnet, die in cis-Lokalisierung auf die Aktivität eines Promotors einwirkt und so die Transkription eines mit diesem Promotor funktionell verknüpften Gens stimuliert. Im Gegensatz zu Promotoren ist die Wirkung der Enhancer positions- und orientierungsunabhängig und können somit vor oder hinter eine Transkriptionseinheit, innerhalb eines Introns oder selbst innerhalb der Kodierregion positioniert werden. Der Enhancer kann dabei sowohl in unmittelbarer Nähe der Transkriptionseinheit als auch in beträchtlichem Abstand zum Promotor lokalisiert sein. Auch eine physikalische und funktionelle Überlappung mit dem Promotor ist möglich. Dem Fachmann sind eine Vielzahl von Enhancern aus verschiedenen Quellen bekannt (und in Datenbanken wie GenBank hinterlegt, z.B. SV40 Enhancer, CMV Enhancer, Polyoma Enhancer, Adenovirus Enhancer) und als eigenständige oder innerhalb von Polynukleotidsequenzen klonierte Elemente verfügbar (z.B. bei ATCC hinterlegt oder aus kommerziellen und individuellen Quellen). Eine Vielzahl von Promotorsequenzen beinhalten auch Enhancersequenzen, wie z.B. der häufig verwendete CMV Promotor. Der humane CMV-Enhancer gehört dabei zu den stärksten bisher identifizierten Enhancern. Ein Beispiel für einen induzierbaren Enhancer ist der Metallothionein-Enhancer, der durch Glucocorticoide oder Schwermetalle stimuliert werden kann.

Grundsätzlich umfassen die regulatorischen Elemente Promotoren, Enhancer, Terminations- und Polyadenylierungssignale und weitere Expressionskontrollelemente. Für die verschiedenen Zelltypen sind sowohl induzierbare als auch konstitutive regulatorische Sequenzen bekannt. ,,Transkriptionsregulatorische Elemente" umfassen gewöhnlich einen Promotor stromaufwärts von der zu exprimierenden Gensequenz, Transkriptionsinitiations- und -terminationsstellen sowie ein Polyadenylierungssignal.

Der Ausdruck ,,Transkriptionsinitiationsstelle" bezieht sich auf eine Nukleinsäure in dem Konstrukt, die der ersten Nukleinsäure entspricht, welche in das primäre Transkript, d.h. den mRNA-Precursor, inkorporiert wird. Die Transkriptionsinitiationsstelle kann mit den Promotorsequenzen überlappen.

Der Ausdruck ,,Transkriptionsterminationsstelle" bezieht sich auf eine Nukleotidsequenz, die normalerweise am 3'-Ende des Gens von Interesse oder des zu transkribierenden Genabschnitts vorhanden ist und die den Abbruch der Transkription durch RNA-Polymerase bewirkt.

Das "Polyadenylierungssignal" ist eine Signalsequenz, welche die Spaltung an einer spezifischen Stelle am 3'-Ende der eukaryontischen mRNA und den posttranskriptionellen Einbau einer Sequenz von etwa 100-200 Adeninnukleotiden (polyA-Schwanz) am gespaltenen 3'-Ende verursacht. Das Polyadenylierungssignal umfasst die Sequenz AATAAA etwa 10-30 Nukleotide stromaufwärts von der Spaltstelle sowie eine stromabwärts gelegene Sequenz. Es sind verschiedene Polyadenylierungselemente bekannt, z.B. tk polyA, SV40 late und early polyA oder BGH polyA (z.B. beschrieben in US 5,122,458).

,,Translationsregulatorische Elemente" umfassen eine Translationsinitiationsstelle (AUG), ein Stoppcodon und ein polyA-Signal für jedes zu exprimierende Polypeptid. Für eine optimale Expression kann es günstig sein, 5'- und/oder 3'-nichttranslatierte Bereiche der zu exprimierenden Nukleinsäuresequenz zu entfernen, hinzuzufügen oder zu ändern, um potentielle zusätzliche ungeeignete Translationsinitiationscodons oder andere Sequenzen, welche die Expression auf dem Niveau der Transkription oder Expression beeinträchtigen können, zu eliminieren. Um die Expression zu fördern, kann man alternativ ribosomale Konsensus-Bindungsstellen unmittelbar stromaufwärts vom Startcodon insertieren. Um ein sekretiertes Polypeptid zu produzieren, enthält das Gen von Interesse gewöhnlich eine Signalsequenz, welche für ein Signalvorläuferpeptid kodiert, die das synthetisierte Polypeptid zu und durch die ER-Membran transportiert. Die Signalsequenz befindet sich oft, jedoch nicht immer, am Aminoterminus des sekretierten Proteins und wird durch Signal-Peptidasen abgespalten, nachdem das Protein durch die ER-Membran geschleust wurde. Die Gensequenz wird gewöhnlich, jedoch nicht notwendigerweise, eine eigene Signalsequenz enthalten. Wenn die native Signalsequenz nicht vorhanden ist, kann in bekannter Weise eine heterologe Signalsequenz eingeführt werden. Dem Fachmann sind zahlreiche solcher Signalsequenzen bekannt und in Sequenzdatenbanken wie GenBank und EMBL hinterlegt.

Genprodukte von Interesse können Proteine/Polypeptide umfassen, z.B. Antikörper, Enzyme, Cytokine, Lymphokine, Adhäsionsmoleküle, Rezeptoren sowie deren Derivate bzw. Fragmente, sind aber nicht auf diese beschränkt. Im Allgemeinen sind alle Polypeptide bedeutsam, die als Agonisten oder Antagonisten wirken und/oder therapeutische oder diagnostische Anwendung finden können.

Der Ausdruck "Polypeptide" wird für Aminosäuresequenzen oder Proteine verwendet und bezeichnet Polymere von Aminosäuren beliebiger Länge. Dieser Ausdruck schließt auch Proteine ein, die posttranslational durch Reaktionen wie beispielsweise Glykosylierung, Phosphorylierung, Acetylierung oder Proteinprozessierung modifiziert werden. Die Struktur des Polypeptids kann, z.B. durch Substitutionen, Deletionen oder Insertion von Aminosäuren, Fusion mit anderen Proteinen, unter Beibehaltung seiner biologischen Aktivität modifiziert werden.

Beispiele für Proteine sind Insulin; Insulin-ähnlicher Wachstumsfaktor (IGF-I oder IGF-II); humanes Wachstumshormon (hGH) und andere Wachstumsfaktoren wie zum Beispiel VEGF, EGF, TGF, beispielsweise TGF-alpha und beta, einschließlich ß1, ß2, ß3, ß4 und ß5; Gewebeplasminogenaktivator (tPA); Erythropoetin (EPO); Thrombopoietin (TBO); Cytokine, beispielsweise Interleukine (IL) wie IL-1, IL-2, IL-3, IL-4, IL-5, IL-6, IL-7, IL-8, IL-9, IL-10, IL-11, IL-12, IL-13, IL-14, IL-15, IL-16, IL-17, IL-18; Interferon (IFN)-alpha, beta, gamma, omega oder tau; Tumornekrosefaktor (TNF) wie z.B. TNF-alpha, beta, gamma, CD40-Ligand, Apo-2-Ligand/TRAIL, DR4, DR5, DcR1, DcR2, DcR3, OPG, Fas Ligand; G-CSF; GM-CSF; M-CSF; MCP-1 und VEGF. Weitere Beispiele sind Gerinnungsfaktoren wie Faktor VII, Faktor VIII, Faktor IX, von Willebrands Faktor; Anti-Gerinnungsfaktoren wie Protein C; Enekephalinase; RANTES (regulated on activation normally T-cell expressed and secreted); humanes Makrophagen Inflammatorisches Protein (MIP-1-alpha); (humanes) Serum Albumin, Zell-Adhäsions-Moleküle, wie LFA-1, Mac-1, p150.95, VLA-4, ICAM-1, ICAM-2, ICAM-3, VCAM, oder (αV/ß3 Integrin einschließlich α oder ß-Untereinheiten; Blutgruppen-Antigene; flk2/3-Rezeptor; OB-Rezeptor; mlp-Rezeptor; CTLA-4; Apo-2L-Rezeptoren wie zum Beispiel Apo-2; *Transforming Growth Factor* (TGF); CD-Proteine, T-Zell Rezeptoren; virale Antigene wie zum Beispiel das gp120 von HIV; Tumor assoziierte Antigene wie zum Beispiel HER2-, HER3- oder HER4-Rezeptor, Rheumatoid Faktoren, beispielsweise NGF-ß oder PDGF; Relaxin-A- oder -B-Kette; Gonadropin; Gonadropin-assoziertes Peptid; Inhibin; Aktivin; ein cytotoxisches T-Lymphozyten-assoziiertes Antigen (CTLA) oder Neurotophin-Faktoren wie zum Beispiel BDNF, Neurotrophin-3, -4, -5 oder-6.

Weitere Beispiele sind monoklonale, polyklonale, multispezifische und einzelkettige *(single chain)* Antikörper und Fragmente davon, wie z.B. Fab, Fab', F(ab')₂, Fc und Fc'-Fragmente, leichte (L) und schwere (H) Immunglobulinketten und deren konstante, variable oder hypervariable Regionen sowie Fv- und Fd-Fragmente (Chamov et al., 1999). Die Antikörper können humanen oder nicht-humanen Ursprungs sein. Auch humanisierte und chimäre Antikörper kommen in Frage.

Fab-Fragmente *(Fragment antigen-binding =* Fab) bestehen aus den variablen Regionen beider Ketten, die durch die angrenzenden konstanten Regionen zusammengehalten werden. Sie können z.B. durch Behandlung mit einer Protease, wie beispielsweise Papain, aus konventionellen Antikörpern erzeugt werden oder aber auch durch DNA-Klonierung. Weitere Antikörperfragmente sind F(ab')₂-Fragmente, die durch proteolytischen Verdau mit Pepsin hergestellt werden können.

Durch Genklonierung können auch verkürzte Antikörperfragmente hergestellt werden, die nur aus den variablen Region der schweren (VH) und der leichten Kette (VL) bestehen. Diese werden als Fv-Fragmente *(Fragment variable =* Fragment des variablen Teils) bezeichnet. Da bei diesen Fv-Fragmenten die kovalente Verbindung über die Cysteinreste der konstanten Ketten nicht möglich ist, werden diese Fv-Fragmente oft anderweitig stabilisiert. Dazu werden die variablen Region der schweren und leichten Kette häufig mittels eines kurzen Peptidfragments von ca. 10 - 30 Aminosäuren, besonders bevorzugt 15 Aminosäuren, miteinander verknüpft.

Auf diese Weise entsteht eine einzelne Polypeptidkette, in der VH und VL durch einen Peptidlinker miteinander verbunden sind. Solche Antikörperfragmente werden auch als *single-chain* Fv-Fragment (scFv) bezeichnet. Beispiele von scFv-Antikörpern sind bekannt und beschrieben, siehe z.B. Huston et al. (1988).

In den vergangenen Jahren wurden verschiedene Strategien entwickelt um multimere scFv-Derivate herzustellen. Die Intention besteht in der Erzeugung von rekombinanten Antikörpern mit verbesserten pharmakokinetischen Eigenschaften und verstärkter Bindungsavidität. Zur Erreichung der Multimerisierung der scFv-Fragmente werden diese als Fusionsproteine mit Multimerisierungsdomänen hergestellt. Als Multimerisierungsdomänen können dabei z.B. die CH3-Region eines IgGs oder Helixstrukturen (*"coiled coil structure")* wie die Leucin-Zipper-Domänen fungieren. In anderen Strategien wird die Interaktion zwischen den VH- und VL-Regionen des scFv-Fragments für eine Multimerisierung genutzt (z.B. Dia-, Tri- und Pentabodies).

Als "Diabody" bezeichnet ein Fachmann ein bivalentes homodimeres scFv-Derivat. Die Verkürzung des Peptidlinkers im scFv-Moleküle auf 5 - 10 Aminosäuren resultiert in der Bildung von Homodimeren durch Überlagerung von VH/VL-Ketten. Die Diabodies können zusätzlich durch eingeführte Disulfidbrücken stabilisiert werden. Beispiele von Diabodies finden sich in der Literatur, z.B. bei Perisic et al. (1994).

Als ,,Minibody" bezeichnet der Fachmann ein bivalentes, homodimeres scFv-Derivat. Es besteht aus einem Fusionsprotein, das die CH3-Region eines Immunglobulins, vorzugsweise IgG, besonders bevorzugt IgG1, als Dimerisierungsregion enthält. Diese verbindet die scFv-Fragmente über eine Hinge-Region, ebenfalls von IgG, und eine Linker-Region. Beispiele solcher Minibodies sind bei Hu et al. (1996) beschrieben.

Mit ,,Triabody" bezeichnet der Fachmann ein trivalentes homotrimeres scFv-Derivat (Kortt et al., 1997). Die direkte Fusion von VH-VL ohne Verwendung einer Linkersequenz führt zur Ausbildung von Trimeren.

Bei den vom Fachmann als Mini-Antikörper bezeichneten Fragmenten, die eine bi-, tri- oder tetravalente Struktur haben, handelt es sich ebenfalls um Derivate von scFv-Fragmenten. Die Multimerisierung wird dabei über di-, tri- oder tetramere "coiled coil"-Strukturen erzielt (Pack et al., 1993 und 1995; Lovejoy et al., 1993).

Zur Selektion von transfizierten Zellen können diese zusätzlich mit einem oder mehreren Selektionsmarkergenen transfiziert werden. In der Literatur sind eine Vielzahl von Selektionsmarkergenen, einschließlich bifunktionaler (positiv/negativ) Marker, beschrieben (siehe z.B. WO 92/08796 und WO 94/28143). Beispiele für Selektionsmarker, die für gewöhnlich in eukaryontischen Zellen verwendet werden, beinhalten die Gene für Aminoglykosid-Phosphotransferase (APH), Hygromycin-Phosphotransferase (HYG), Dihydrofolat-Reduktase (DHFR), Thymidinkinase (TK), Glutamin-Synthetase, Asparagin-Synthetase und Gene, die Resistenz gegenüber Neomycin (G418), Puromycin, Histidinol D, Bleomycin, Phleomycin und Zeocin vermitteln. Diese Gene können zusammen mit dem Gen von Interesse oder aber separat in die Zelle eingebracht werden. Vorzugsweise werden sie hierbei ebenfalls über Expressionsvektoren in die Zellen eingebracht. Entsprechend modifizierte Zellen können nun in Anwesenheit von einem oder mehreren geeigneten Selektionsmittel kultiviert werden, die selektiv Zellen im Wachstum bevorzugen, die ein entsprechendes Selektionmarkergen enthalten und exprimieren.

Eine Selektion von transfizierten Zellen ist auch durch fluoreszenzaktivierte Zellsortierung (FACS) möglich, beispielsweise über bakterielle β-Galaktosidase, Zelloberflächenmarker oder fluoreszierende Proteine. Die fluoreszierenden Proteine ermöglichen darüber hinaus auch eine FACS-basierte Isolierung von einzelnen Säugerzellen. Entsprechend detektierte Zellen lassen sich automatisch als Einzelzelle oder zu mehreren Zellen, z.B. mit Hilfe eines Lasers, beispielsweise mit einem Argon-Laser (488nm) und beispielsweise mit einem mit einer Autoclone-Einheit bestückten Flow Cytometer (Coulter EPICS Altra, Beckman-Coulter, Miami, FL, USA), in Kulturgefäße ablegen. Nach einer bevorzugten Ausführungsform werden lediglich eine (1) einzelne oder höchsten zwei Zellen in ein mit autologen feeder-Zellen bestücktes Zellkulturgefäß auf diese Weise abgelegt. Besonders vorteilhaft ist die entsprechende Ablage von jeweils nur einer (1) einzelnen Zelle. Darüber hinaus kann eine Sortierung über magnetische Beads erfolgen. Dazu erfolgt eine Markierung der Zellen über z.B. Antikörper, die an Magnetbeads gekoppelt sind. Dies erlaubt eine Sortierung von Zellen nach definierten Eigenschaften.

Besonders vorteilhaft ist die FACS-basierte Isolierung von Zellklonen, die nach einem der hier beschriebenen Verfahren abgelegt wurden und ein fluoreszierendes Protein und ein Gen von Interesse co-exprimieren. Vorzugsweise sind die Expression des fluoreszierenden Proteins und des Gens von Interesse funktionell miteinander verknüpft. Eine solche funktionelle Verknüpfung besteht beispielsweise, wenn sich beide Gene in einer engen räumlichen Anordnung befinden, so dass die Expressionsraten beider Gene miteinander korrelieren, beispielsweise nach transienter oder stabiler Transfektion einer Wirtszelle. Eine solche funktionelle Verknüpfung lässt sich auch beispielsweise durch die Verwendung von sog. IRES-Elementen *(= internal ribosome entry site)* oder durch RNA-Splicing erreichen, wobei beide Gene (Gen von Interesse und Gen des fluoreszierenden Proteins) als bicistronische mRNA synthetisiert werden. Auf diese Weise besteht ein direkte Korrelation zwischen der Expressionsrate des fluoreszierenden Proteins und des Gens von Interesse. Die entsprechenden Zellklone, die eine hohen Expression an fluoreszierendem Protein zeigen verfügen in Folge der funktionellen Verknüpfung auch über eine hohe Expressionsrate des Gens von Interesse.

Bei dem fluoreszierenden Protein kann es sich z.B. um ein grün, blaugrün, blau, gelb oder andersfarben fluoreszierendes Protein handeln. Ein spezielles Beispiel ist das grün fluoreszierende Protein (GFP) aus *Aequorea victoria* oder *Renilla reniformis* und daraus entwickelte Mutanten; siehe z.B. Bennet et al. (1998); Chalfie et al. (1994); WO 01/04306 und die dort zitierte Literatur. Weitere fluoreszierende Proteine und dafür kodierende Gene sind in WO 00/34318, WO 00/34326, WO 00/34526 und WO 01/27150 beschrieben, die durch Bezugnahme hierin inkorporiert werden. Bei diesen fluoreszierenden Proteinen handelt es sich um Fluorophore von nichtbiolumineszierenden Organismen der Spezies Anthozoa, beispielsweise von *Anemonia majano, Clavularia sp., Zoanthus sp. I, Zoanthus sp.* II, *Discosoma striata, Discosoma sp.* ,,red", *Discosoma sp.* "green", *Discosoma sp.* "Magenta", *Anemonia sulcata.* Die eingesetzten Fluoreszenzproteine können aus den Wildtyp-Proteinen, natürlichen oder gentechnologisch hergestellten Mutanten und -varianten, deren Fragmente, Derivate oder z.B. mit anderen Proteinen oder Peptiden fusionierte Varianten bestehen. Die eingebrachten Mutationen können dabei beispielsweise das Exzitations- oder Emissionsspektrum, die Chromophorenbildung, den Extinktionskoeffizienten oder die Stabilität des Proteins verändern. Durch Codon-Optimierung kann zudem die Expression in Säugerzellen oder anderen Spezies verbessert werden. Erfindungsgemäß kann das fluoreszierende Protein auch in Fusion mit einem Selektionsmarker, vorzugsweise mit einem amplifzierbaren Selektionsmarker wie beispielsweise der Dihydrofolat-Reduktase (DHFR), eingesetzt werden.

Der Selektionsschritt kann an Zellpopulationen oder mit bereits vorsortierten Zellpopulationen/Zellklonen durchgeführt werden. Es können ein oder mehrere, vorzugsweise ein (1), zwei, drei oder vier Zellen pro Zellkulturgefäß abgelegt werden. Vorzugweise erfolgt die Zellablage in serumfreies Medium, besonders bevorzugt in chemisch definiertes Medium, in Gegenwart von autologen feeder-Zellen. Auf geeignete Medien sowie auf erfindungsgemäße Verfahren zur Zellablage unter Verwendung von autologen *feeder-Zellen* wird an anderen Stelle dieser Anmeldung im Detail eingegangen. Grundsätzlich können auch zwei oder mehr Sortierungsschritte durchgeführt werden, wobei zwischen den einzelnen Sortierungsschritten die Zellen über einen bestimmten Zeitraum, z.B. etwa zwei Wochen als Pools, in einem entsprechend geeigneten Medium kultiviert und vermehrt werden.

Zur Produktion eines oder mehrerer Genprodukte von Interesse in den reklonierten Zellen werden die reklonierten Zellen vorzugsweise in einem serumfreien Kulturmedium und in Suspensionskultur unter Bedingungen gezüchtet, die eine Expression des Gens von Interesse erlauben. Steht das Gen von Interesse beispielsweise unter der Kontrolle konstitutiver Promotoren, so ist eine Zugabe von speziellen Induktoren nicht erforderlich. Steht die Expression des Gens von Interesse beispielsweise unter der Kontrolle induzierbarer Promotoren, so ist dem Zellkulturmedium ein entsprechender Induktor in ausreichender aber nicht toxischer Konzentration zuzugeben. Die Zellen können durch mehrfaches Subpassagieren beliebig expandiert und in entsprechend geeignete Zellkulturgefäße überführt werden. Die Produktion des/der Genprodukte erfolgt entweder als zelluläres, membrangebundenes oder als sekretorisches Produkt.

Das Produkt von Interesse wird vorzugsweise als sekretiertes Genprodukt aus dem Zellkulturmedium gewonnen. Bei Expression eines Proteins oder Polypeptids ohne Sekretionssignal kann das Genprodukt aber auch aus Zelllysaten isoliert werden. Um ein reines, homogenes Produkt zu erhalten, das im Wesentlichen frei ist von anderen rekombinanten Proteinen und Wirtszellproteinen, werden übliche Reinigungsschritte durchgeführt. Hierzu entfernt man häufig zunächst Zellen und Zelltrümmer aus dem Kulturmedium oder Lysat. Das gewünschte Genprodukt kann dann von kontaminierenden löslichen Proteinen, Polypeptiden und Nukleinsäuren befreit werden, z.B. durch Fraktionierung an Immunaffinitäts- und Ionenaustauschsäulen, Ethanolfällung, Umkehrphasen-HPLC oder Chromatographie an Sephadex, Silica oder Kationenaustauscherharzen wie DEAE. Methoden, die zur Aufreinigung eines von rekombinanten Wirtszellen exprimierten heterologen Proteins führen, sind dem Fachmann bekannt und sind in der Literatur beschrieben, z.B. bei Harris et al. (1995) und Scopes (1988).

Die vorliegende Erfindung stellt erstmals Hamsterzellen als feeder-Zellen gemäß den Ansprüchen zur Verfügung. Aus diesem Grund betrifft die vorliegende Erfindung auch die Verwendung einer Hamsterzelle als feeder-Zelle gemäß den Ansprüchen. Verfahren zur Herstellung entsprechender feeder-Zellen sind in den Ausführungsbeispielen näher beschrieben. Die entsprechenden Hamster- feeder-Zellen lassen sich grundsätzlich durch dem Fachmann bekannte chemische oder physikalische Verfahren, beispielsweise durch Behandlung mit Mitomycin C (Azirino[2',3':3,4]pyrrolo[1,2-a]indole-4,7-dione,6-amino-8-[[(aminocarbonyl)oxy] methyl]-1,1 a,2,8,8a,8b-hexahydro-8a-methoxy-5-methyl-, [1aR-(1a.alpha., 8.beta., 8a.alpha., 8b.alpha.)]- (9Cl) (Butcher et al, 1988)) oder durch Bestrahlung mit ¹³⁷Cs herstellen. Nach einer bevorzugten Ausführungsform betrifft die vorliegende Erfindung von daher die Verwendung von chemisch oder physikalisch inaktivierten feeder-Zellen. Der Begriff "inaktiviert" meint in diesem Zusammenhang, dass die Zellen in ihrer Teilungsfähigkeit eingeschränkt sind, vorzugsweise diese verloren haben, darüber hinaus aber noch vital sind. Dies meint, dass die Zellen noch Stoffwechselaktivitäten wie beispielsweise die Synthese und Sekretion von Wachstumsfaktoren für einen bestimmten Zeitraum aufweisen, vorzugsweise zumindest für ein bis zwei Wochen nach Inaktivierung. Nach einer bevorzugten Ausführungsform handelt es sich bei den entsprechenden feeder-Zellen um *feeder-*Zellen die an serumfreie Kulturbedingungen adaptiert sind. Nach einer weiter bevorzugten Ausführungsform der Erfindung handelt es sich bei den feeder-Zellen um CHO-oder BHK- Zellen sowie um Abkömmlinge/Derivate dieser Zelllinien.

Darüber hinaus lassen sich alle in dieser Anmeldung erwähnten Hamster- Zellen nach entsprechender Inaktivierung als feeder-Zellen verwenden.

Beispiele für serumfreie, proteinfreie oder chemisch definierte Medien stellen beispielsweise die im Handel erhältlichen Medien Ham's F12 (Sigma, Deisenhofen, DE), RPMI-1640 (Sigma), Dulbecco's Modified Eagle's Medium (DMEM; Sigma), Minimal Essential Medium (MEM; Sigma), Iscove's Modified Dulbecco's Medium (IMDM; Sigma), CD-CHO (Invitrogen, Carlsbad, Ca., USA), CHO-S-SFMII (Invitrogen), serumfreies CHO-Medium (Sigma) und proteinfreies CHO-Medium (Sigma) dar. Jedes dieser Medien kann gegebenenfalls mit verschiedenen Verbindungen ergänzt werden, z.B. Hormonen und/oder anderen Wachstumsfaktoren (z.B. Insulin, Transferrin, epidermalem Wachstumsfaktor, Insulin-ähnlichem Wachstumsfaktor), Salzen (z.B. Natriumchlorid, Calcium, Magnesium, Phosphat), Puffern (z.B. HEPES), Nukleosiden (z.B. Adenosin, Thymidin), Glutamin, Glukose oder anderen äquivalenten Nährstoffen, Antibiotika und/oder Spurenelementen. Handelt es sich bei den vermehrungsfähigen Zellen um rekombinante Zellen, die einen oder mehrere Selektionsmarker exprimieren, so kann dem Medium auch ein oder mehrere geeignete Selektionsmittel, z.B. Antibiotika zugefügt werden.

### BEISPIELE

### Abkürzunaen

- ATCC: American Type Culture Collection
- BHK: Baby Hamster Kidney
- ⁶⁰Co: Kobalt Isotop 60
- ¹³⁷Cs: Cäsium Isotop 137
- CHO: Chinese Hamster Ovary
- CMV: Cytomegalievirus
- DE: Deutschland
- DEAE: Diethylaminoethyl-
- DMSO: Dimethylsulfoxide
- DNA: Desoxyribonukleinsäure
- FACS: Fluorescence-activated cell sorter
- FITC: Fluorescein-Isothiocyanat
- Gy: Gray
- HBSS: Hank's balanced salt solution
- HPLC: High performance liquid chromatograhy
- mRNA: Messenger Ribonukleinsäure
- NSO: Maus-Hybridoma-Zelle
- polyA: Polyadenylierungssequenz
- Sp2/0: Maus-Hybridoma-Zelle
- SV40: Simian Virus No.40

### Methoden

### 1. Kultivierung der Zellen

Die Zellen CHO-DG44/dhfr -/- (Urlaub et al., 1983) wurden permanent als Suspensionszellen in serum-freiem und mit Hypoxanthin und Thymidin supplementiertem CHO-S-SFMII Medium (Invitrogen GmbH, Karlsruhe, DE) in Zellkulturflaschen bei 37°C in feuchter Atmosphäre und 5% CO₂ kultiviert. Die Zellzahlen sowie die Viabilität wurden mit einem CEDEX Cell Counter (Innovatis, DE) oder durch Trypanblau-Färbung bestimmt und die Zellen dann in einer Konzentration von 1 - 3 ×10⁵/mL eingesät und alle 2 - 3 Tage passagiert. Für die Einzelzellklonierung wurden rekombinante CHO-DG44/dhfr^{-/-} verwendet, die ein fluoreszentes Protein (beispielsweise ZS-Green aus *Zoanthus sp.)* bzw. ein fluoreszentes Protein und einen humanen bzw. einen humanisierten monoklonalen Antikörper exprimieren. Die Kultivierung klonierter rekombinanter Zellen erfolgte analog zu diesen Zellen. Als Medium wurde ebenfalls CHO-S-SFM-II Medium (Invitrogen GmbH, Karlsruhe, DE) ohne Hypoxanthin und Thymidin verwendet.
Die Zellen BHK lassen sich permanent als Suspensionszellen in serum-freiem Opti Pro SFM Medium (Invitrogen GmbH, Karlsruhe, DE) in Zellkulturflaschen bei 37°C in feuchter Atmosphäre und 5% CO₂ kultivieren. Die Zellzahlen sowie die Viabilität kann mit einem CEDEX Cell Counter (Innovatis, DE) oder durch Trypanblau-Färbung bestimmt werden, wobei die Zellen dann in einer Konzentration von 1 - 3 x10⁵/mL eingesät und alle 2 - 3 Tage passagiert werden. Die Kultivierung klonierter Zellen erfolgt analog den BHK-Zellen.
Die Zellen NSO lassen sich permanent als Suspensionszellen in serum-freiem Hybridoma Medium, Animal component free Medium (Sigma, Aldrich, St. Louis, USA) in Zellkulturflaschen bei 37°C in feuchter Atmosphäre und 5% CO₂ kultivieren. Die Zellzahlen sowie die Viabilität kann mit einem CEDEX Cell Counter (Innovatis, DE) oder durch Trypanblau-Färbung bestimmt werden und die Zellen werden dann in einer Konzentration von 1 - 3 x10⁵/mL eingesät und alle 2 - 3 Tage passagiert. Die Kultivierung klonierter Zellen erfolgt analog den Zellen NSO. Als Medium wird Hybridoma Medium, Animal component free Medium (Sigma, Aldrich, St. Louis, USA) verwendet.

### 2. Herstellung von feeder-Zellen durch Bestrahlung

Serum- und proteinfrei wachsende suspendierte CHO-Grundzellen (nicht transfizierte Zellen), wurden bei 180g für 10 Minuten abzentrifugiert und auf eine Zellkonzentration von 1 x 10⁶/mL in HBSS (Hank's balanced salt solution) eingestellt Danach erfolgte die Bestrahlung der Zellen mit einer radioaktiven Bestrahlungsquelle (Cs137-Strahler, Gammacell 2000, Fa. Molsgaard Medical A/S, Dänemark) mit einer Energiedosisleistung von 4Gy/min. Mit einer Bestrahlungszeit zwischen 5min und 125min ergab sich eine Energiedosis zwischen 20 und 500 Gy. Nach der Bestrahlung wurden die Zellen mit ca. 2000 Zellen/well (= Kulturgefäß) in 96-Loch-Mikrotiterplatten in dem für die Zelle spezifischen CHO-S-SFMII Medium eingesät und bei ca. 37°C und 5% CO₂ in Brutraumatmosphäre gelagert. Das Verfahren wird entsprechend mit BHK- und NSO-Zellen durchgeführt, wobei die feeder-Zellen in dem jeweils für die Zellen spezifischen Medium gehalten/eingesät werden.

### 3. Kryokonservierung von feeder-Zellen

Die entsprechend hergestellten feeder-Zellen können bei unter - 150°C kryokonserviert werden. Die Kryokonservierung wird mit Hilfe eines programmierbarem Einfriergerät im jeweiligen Zellkulturmedium durchgeführt (Consarctic BV-25, Consarctic, Schöllkrippen, DE). Als Kryoprotektant wird den Medien 10% (v/v) DMSO zugesetzt. Die Einfrierrate zwischen 0°C und -20°C beträgt 1 °C/min, anschließend erfolgt die weitere Temperaturabsenkung mit 0.4°C/min. Nach erfolgtem Einfrierprozess werden die feeder-Zellen in der Gasphase in flüssigem Stickstoff kryokonserviert.

### 4. Automatisierte Zellablage

Die automatische Zellablage (Einzel- oder Mehrzellablage) wird mit einem mit Argon-Laser (488nm) bestückten Flow Cytometer (Coulter EPICS Altra (Fa. Beckman-Coulter, Miami, FL, USA) mittels Autoclone-Einheit durchgeführt. Die Zellen werden hierbei in der exponentiellen Wachstumsphase abzentrifugiert und auf eine Zellkonzentration von 1 - 1,5 x10⁷/mL in HBSS aufgenommen. Anschließend werden die Zellen mit der "Hypersort-Option" mit einer Geschwindigkeit von 8000-12000 Zellen/Sekunde nach ihrer Lage im Streulicht sortiert. Zellen, die ein Fluoreszenzprotein exprimieren, lassen sich alternativ gemäß ihrer Fluoreszenzintensität in Bezug auf das intrazellulär exprimierte Fluoreszenzprotein sortieren. Die Zellen werden jeweils einzeln in mit feeder-Zellen bestückten 96-Loch Mikrotiterplatten abgelegt. Die Ablage von BHK-Zellen erfolgt beispielsweise in OptiPro SFM Medium (Invitrogen GmbH, Karlsruhe, DE). Die sortierten NSO-Zellen werden beispielhaft in Hybridoma Medium, Animal component free Medium (Sigma, Aldrich, St. Louis, USA) abgelegt.
Bei der Sortierung von CHO-Zellen erfolgte die Zellablage in CHO-S-SFM-11 (Invitrogen GmbH, Karlsruhe, DE). Abgelegt wurde eine rekombinante CHO-DG-44-Zelle, die einen humanen bzw. einen humanisierten monoklonalen Antikörper sowie das ZS-Green aus *Zoanthus sp.* co-exprimierte. Die Zellsortierung erfolgte wie oben beschrieben mit einem Argon-Laser bei 488nm.

### 5. Bestimmung der Produktivität rekombinant exprimierter Genprodukte (für die verwendeten mAk-exprimierenden CHO-DG-44)

Die Quantifizierung der Antikörpertiters in den Überständen von stabil transfizierten CHO-DG44 Zellen, die einen humanen bzw. humanisierten monoklonalen Antikörper exprimieren, erfolgte mittels ELISA nach Standardprotokollen (Ausubel et al., 1994, updated), wobei zum einen ein Ziege anti-Human IgG Fc-Fragment (Dianova, Hamburg, DE) und zum anderen ein AP-konjugierter Ziege anti Human Kappa light chain Antikörper (Sigma) eingesetzt wurde. Als Standard diente der gereinigte Antikörper. Die Produktivitäten (pg/Zelle/Tag) wurden nach der Formel pg/((Ct-Co) t / In (Ct-Co)) berechnet, wobei Co und Ct die Zellzahl bei Aussaat bzw. Ernte und t die Kultivierungsdauer angibt.

### Beispiel 1: Einfluss der Energiedosis bei der Herstellung der feeder-Zellen auf die Reklonierungseffizienz von CHO-DG-44 Zellen

Um den Einfluss der Energiedosis bei der Herstellung der *feeder-Zellen* auf die Reklonierungseffizienz zu untersuchen, wurden die CHO-DG-44 Zellen wie unter Methoden ,,Kultivierung der Zellen" beschrieben kultiviert. Die Herstellung der *feeder-*Zellen erfolgte wie in Methoden ,,Herstellung von feeder-Zellen durch Bestrahlung" beschrieben mit einer Energiedosis von jeweils 20 Gy, 50 Gy, 100 Gy, 200 Gy und 500 Gy. Nach der Bestrahlung wurden die *feeder-Zellen* in 96-Loch-Mikrotiterplatten mit einer Zellzahl von ca. 2000 Zellen/well eingesät und in Brutraumatmosphäre gelagert. Im Anschluss erfolgte eine automatisierte Einzelablage mit einer rekombinanten CHO-DG-44 Zelle, die ein fluoreszentes Protein exprimierte, wie unter Methoden ,,Automatisierte Einzelzellablage" beschrieben. Hierbei wurde pro Well jeweils eine (1) einzelne Zelle zu den *feeder-Zellen* abgelegt. Als Zielgröße für die Reklonierungseffizienz diente die Anzahl der positiven Wells, d.h. die Wells, in denen sich Klone befanden die nach einer Inkubationszeit von drei Wochen zu einer Zellpopulation heranwuchsen. Die erzielten Reklonierungseffizienzen lagen zwischen 40 und 70% für die Reklonierung von rekombinanen CHO-DG-44 Zellen (vgl. Figur 1).

### Beispiel 2: Homogenität der Reklonierung von Antikörper-exprimierenden CHO-DG-44 Zellen

Zum Vergleich der Homogenität der Zellklone wurden rekombinante Antikörperexprimierende CHO-DG-44 Zellen zum einen nach der Standardmethode "Limited dilution" abgelegt und kloniert und zum andern mittels der hier beschriebenen Einzelzellablage in Gegenwart autologer *feeder-Zellen* abgelegt und kloniert (vgl. Figur 2). Dazu wurden jeweils 6 Zellpools von transfizierten Antikörper-exprimierenden CHO-DG-44 Zellen nach der Limited-dilution-Methode und parallel nach der automatisierten Einzelzellablage, wie unter ,,Kultivierung der Zellen" beschrieben, kultiviert und anschließend wie unter ,,Automatisierte Einzellablage" beschrieben rekloniert. Die daraus entstanden Klone wurden wie unter "Kultivierung der Zellen" beschrieben kultiviert und über die Dauer von drei Passagen der Produkttiter nach der Methode "Bestimmung der Produktivität rekombinant exprimierter Genprodukte" bestimmt. Der aus diesen drei Passagen resultierende Mittelwert wurde zur Erstellung der Grafik herangezogen.

### Beispiel 3: Hochdurchsatzverfahren zur Generierung von Hochtiter-Zellklonen durch Kombination der Expression fluoreszierender Proteinen mit FACS basiertem Zellsortieren und FACS basierter Einzelzellablage

Durch Transfektion von CHO DG44-Zellen mit einem Expressionsvektor, der die bicistronische Expression eines Produktgens (rekombinanter Antikörper) und eines fluoreszierenden Proteins (ZS-Green aus *Zoanthus sp.)* kodiert, wurden Zellpools enthalten, die sowohl den Antikörper als auch das fluoreszierende Protein co-exprimieren. Diese Zellpools wurden nach der bereits beschriebenen Methode in Gegenwart autologer CHO *DG44-Feederzellen* einzeln in Mikrotiterplatten abgelegt und kultiviert. Die Ablage der Klone wurde mittels drei unterschiedlicher Kriterien durchgeführt.
A.) Ablage aller lebenden Zellen
B.) Ablage der 20% am stärksten floureszierenden Zellen
C.)Ablage der 5% am stärksten floureszierenden Zellen

Die erhaltenen Klone wurden danach in eine 24-well-Makrotiterplatte überführt und drei Passagen wie unter ,,Kultivierung der Zellen" beschrieben, kultiviert. Am Ende jeder Passage wurde der Titer des Antikörpers im Überstand nach der Methode "Bestimmung der Produktivität rekombinant exprimierter Genprodukte" bestimmt. Der aus diesen drei Passagen resultierende Mittelwert wurde zur Erstellung der Grafik herangezogen. Hierzu wurden die Anzahl der erhaltenen Klone über definierte Titerklassen aufgetragen und einer Normalverteilung angepasst.

### LITERATUR

Ausubel, F.M. et al., Current Protocols in molecular biology. New York : Greene Publishing Associates and Wiley-Interscience. 1994 (updated)
Bennett, R.P. et al., BioTechniques 1998, 24, 478 - 482
Brodin et al., Journal of Immunological Methods, 1988, 2, 245-251
Butcher et al., Journal of Immunological Methods, 1988, 107, 245-251
Chalfie, M. et al., Science 1994, 263, 802 - 805
Chamov, S.M. et al., Antibody Fusion Proteins, Wiley-Liss Inc., 1999
Freshney RI, (eds.), Animal cell culture: A Practical Approach, IRL Press, Oxford 1986
Gossen, M. et al., Curr Opi Biotech 1994, 5, 516 - 520
Grigoriev et al., Analytical Biochemistry, 1996, 236, 250-254
Harris et al., Protein Purification : A Practical Approach, Pickwood and Hames, eds., IRL Press, Oxford, 1995
Hlinak A. et al., Folia Biologica (Praha) 1987, 34, 105.117
Hu, S. et al., Cancer Res. 1996, 56 (13), 3055 - 3061
Huston, C. et al., Proc Natl Acad Sci USA 1988, 85 (16), 5879 - 5883
Kaufmann H. et al., Biotechnology and Bioengineering 2001, 72, 592-602
Kortt, A.A. et al., Protein Engineering 1997, 10 (4), 423 - 433
Long et al., Journal of Immunological Methods, 1986, 86, 89-93
Lovejoy, B. et al., Science 1993, 259, 1288 - 1293
Meng YG., et al., Gene 2000, 242, 201-207
Morgan (eds.), Kultur tierischer Zellen, Spektrum Akademischer Verlag, Heidelberg, 1994
Mueller et al., Biotechnology and Bioengineering 1999, 65, 529-532
Pack, P. et al., Biotechnology 1993, 11, 1271 - 1277
Pack, P. et al., J Mol Biol 1995, 246 (11), 28 - 34
Peng et al., Biotechnology and Bioengineering, 1996, 50, 479-492
Perisic, O. et al., Structure 1994, 2, 1217 - 1226
Pintus et al., Journal of Immunological Methods, 1983, 61, 195-200
Puck, TT et al., J Exp Med 1958, 108, 945 - 956
Rexroad et al., Molecular Reproduction and Development, 1997, 48, 238-245
Rheinwald, JG and Green, Cell 1975, 6, 331-344
Sambrook, J., Fritsch, E.F. & Maniatis, T.,Molecular Cloning: A Laboratory Manual Cold Spring Harbor Laboratory, Cold Spring Harbor, New York, 1989
Sanchez et al., Journal of Imunological Methods, 1991, 145, 193-197
Scopes, R., Protein Purification, Springer Verlag, 1988
Shapiro, Practical Flow Cytometry, 1995, John Wiley ans Sons, Inc., New York, 3rd. edition
Shneyour et al., Plant Science Letters, 1984, 33, 293-302
Strick-Marchand et al., Mechanisms of Development, 2003, 120, 89-98
Urlaub, G. et al., Cell 1983, 33, 405 - 412
Wee Eng Lim et al., Proteomics 2002, 2, 1187-1203
Williams RL., et al., Nature 1988, 336, 684-687

## Patentansprüche

1. Verwendung einer Hamsterzelle als *feeder*-Zelle zur Vermehrung/ Klonierung von in Suspension wachsenden autologen Hamsterzellen unter serumfreien Bedingungen, **dadurch gekennzeichnet, dass** die als *feeder*-Zelle verwendete Hamsterzelle an serumfreie Kulturbedingungen adaptiert ist und ebenfalls in Suspension gehalten wird, wobei die als *feeder*-Zelle verwendete Hamsterzelle physikalisch oder chemisch inaktiviert wird.

2. Verwendung nach Anspruch 1 **dadurch gekennzeichnet, dass** die zu vermehrende Zelle eine Zelllinie ist, die für die Produktion von Biopharmazeutika von Bedeutung ist.

3. Verwendung nach Anspruch 1 oder 2 **dadurch gekennzeichnet, dass** es sich um eine Verwendung bei einem Verfahren zur Reklonierung von Zellen handelt.

4. Verwendung nach Anspruch 1 **dadurch gekennzeichnet, dass** die als *feeder*-Zelle verwendete Hamsterzelle durch Bestrahlung inaktiviert wird.

5. Verwendung nach Anspruch 4 **dadurch gekennzeichnet, dass** die Energiedosis der Strahlung 20-500 Gy beträgt.

6. Verwendung nach einem der Ansprüche 1 bis 5 **dadurch gekennzeichnet, dass** es sich bei der als *feeder*-Zelle verwendeten Hamsterzelle um eine Zelle gemäß folgender Tabelle handelt:
| Zelllinie | Hinterlegungsnummer |
|---|---|
| BHK21 | ATCC CCL-10 |
| BHK TK- | ECACC No. 85011423 |
| HaK | ATCC CCL-15 |
| 2254-62.2 (BHK-21-Derivat) | ATCC CRL-8544 |
| CHO | ECACC No. 8505302 |
| CHO-K1 | ATCC CCL-61 |
| CHO-DUKX | ATCC CRL-9096 |
| (= CHO duk⁻, CHO/dhfr⁻) | |
| CHO-DUKX B1 | ATCC CRL-9010 |
| CHO-DG44 | Urlaub et al., Cell 33[2], 405-412, 1983 |
| CHO Pro-5 | ATCC CRL-1781 |
| V79 | ATCC CCC-93 |
| B14AF28-G3 | ATCC CCL-14 |
| CHL | ECACC No. 87111906 |
oder ein Derivat oder ein Abkömmling einer solchen Zelle/Zelllinie.

7. Verwendung nach Anspruch 6 **dadurch gekennzeichnet, dass** es sich bei der als *feeder*-Zelle verwendeten Hamsterzelle um eine CHO oder BHK-Zelle handelt, oder um Abkömmlinge/Derivate dieser Zelllinien.

8. Verwendung nach Anspruch 7 **dadurch gekennzeichnet, dass** es sich bei der als *feeder*-Zelle verwendeten Hamsterzelle um eine CHO Zelle handelt.

9. Verwendung nach Anspruch 8 **dadurch gekennzeichnet, dass** es sich bei der CHO Zelle um eine CHO DG44 Zelle handelt.

## Claims

1. Use of a hamster cell as a feeder cell for replicating/cloning autologous hamster cells grown in suspension under serum-free conditions, **characterised in that** the hamster cell used as a feeder cell is adapted to serum-free culture conditions and is also kept in suspension, the hamster cell used as the feeder cell being physically or chemically inactivated.

2. Use according to claim 1, **characterised in that** the cell to be replicated is a cell line that is of importance for the production of biopharmaceuticals.

3. Use according to claim 1 or 2, **characterised in that** it relates to use in a method of recloning cells.

4. Use according to claim 1, **characterised in that** the hamster cell used as a feeder cell is inactivated by irradiation.

5. Use according to claim 4, **characterised in that** the energy dose of the radiation is 20-500 Gy.

6. Use according to one of claims 1 to 5, **characterised in that** the hamster cell used as a feeder cell is a cell as shown in the following Table:
| Cell line | Accession number |
|---|---|
| BHK21 | ATCC CCL-10 |
| BHK TK- | ECACC No. 85011423 |
| HaK | ATCC CCL-15 |
| 2254-62.2 (BHK-21 derivative) | ATCC CRL-8544 |
| CHO | ECACC No. 8505302 |
| CHO-K1 | ATCC CCL-61 |
| CHODUKX | ATCC CRL-9096 |
| (= CHO duK⁻, CHO/dhfr⁻) | |
| CHODUKX B1 | ATCC CRL-9010 |
| CHO-DG44 | Urlaub et al., Cell 33[2], 405-412, 1983 |
| CHO Pro-5 | ATCC CRL-1781 |
| V79 | ATCC CCC-93 |
| B14AF28-G3 | ATCC CCL-14 |
| CHL | ECACC No. 87111906 |
or a derivative or descendant of such a cell or cell line.

7. Use according to claim 6, **characterised in that** the hamster cell used as the feeder cell is a CHO or BHK cell, or descendants or derivatives of these cell lines.

8. Use according to claim 7, **characterised in that** the hamster cell used as the feeder cell is a CHO cell.

9. Use according to claim 8, **characterised in that** the CHO cell is a CHO DG44 cell.

## Revendications

1. Utilisation d'une cellule de hamster comme cellule nourricière pour la multiplication/ le clonage de cellules de hamster autologues croissant en suspension dans des conditions sans sérum, **caractérisée en ce que** la cellule de hamster utilisée comme cellule nourricière est adaptée aux conditions de culture sans sérum et est également maintenue en suspension, la cellule de hamster utilisée comme cellule nourricière étant physiquement ou chimiquement inactivée,

2. Utilisation selon la revendication 1, **caractérisée en ce que** la cellule à multiplier est une lignée cellulaire qui est importante pour la production d'agents biopharmaceutiques.

3. Utilisation selon la revendication 1 ou 2, **caractérisée en ce qu'**il s' agit d'une utilisation dans un procédé de reclonage de cellules.

4. Utilisation selon la revendication 1, **caractérisée en ce que** la cellule de hamster utilisée comme cellule nourricière est inactivée par irradiation.

5. Utilisation selon la revendication 4, **caractérisée en ce que** la dose d'énergie de rayonnement est de 20 à 500 Gy.

6. Utilisation selon l'une des revendications 1 à 5, **caractérisée en ce que** la cellule de hamster utilisée comme cellule nourricière est une cellule selon le tableau suivant :
| Lignée cellulaire | N° d'enregistrement |
|---|---|
| BHK21 | ATCC CCL-10 |
| BHK TK⁻ | ECACC n° 85011423 |
| HaK | ATCC CCL-15 |
| 2254-62.2 (dérivé de BHK- 21) | ATCC CRL-8544 |
| CHO | ECACC n° 8505302 |
| CHO-K1 | ATCC CCL-61 |
| CHO-DUKX (= CHO duk⁻, CHO/ dhfr⁻) | ATCC CRL-9096 |
| CHO-DUKX B1 | ATCC CRL-9010 |
| CHO-DG44 | Urlaub et coll., Cell 33[2], 405-412, 1983 |
| CHO Pro-5 | ATCC CRL-1781 |
| =V79 | ATCC CCC-93 |
| B14AF28-G3 | ATCC CCL-14 |
| CHL | ECACC n° 87111906 |
ou un dérivé ou un descendant de cette cellule/ lignée cellulaire.

7. Utilisation selon la revendication 6, **caractérisée en ce que** la cellule de hamster utilisée comme cellule nourricière est une cellule CHO ou une cellule BHK ou un descendant/ dérive de ces lignées cellulaires.

8. Utilisation selon la revendication 7, **caractérisée en ce que** la cellule de hamster utilisée comme cellule nourricière est une cellule CHO.

9. Utilisation selon la revendication 8, **caractérisée en ce que** la cellule CHO est une cellule CHO DG44,
